# EUROPEAN PATENT APPLICATION

(11) **EP 3 806 099 A1**
(43) Date of publication of application: **14.04.2021**
(21) Application number: 18919719.7
(22) Date of filing: 24.05.2018
(51) Int. Cl.: G16H 10/00

(54) **SERVER DEVICE AND SERVICE PROVIDING SYSTEM**

(71) Applicant: Toyo Kohan Co., Ltd., Tokyo 141-8260 (JP); Ilac Co., Ltd., Tsukuba-shi, Ibaraki, 305-8550 (JP)
(72) Inventor: SAITO Masahiro, Tokyo 102-8447 (JP); OKAMURA Hiroshi, Tokyo 102-8447 (JP); SATO Takaaki, Tsukuba-shi, Ibaraki 305-8550 (JP)
(74) Representative: Dietz, Christopher Friedrich
(86) International application number: PCT/JP2018/019985
(87) International publication number: WO 2019/224971

(57) **Abstract**

Provided is a technique that allows extensively collecting genome information (specimens) from general persons other than patients, and allows effective utilization of the genome information. A server device provides a genome related service and includes a storage device that stores a program to provide the service and a processor that reads the program from the storage device and executes the program. The processor executes: a process that generates information that can be exchanged for value for predetermined settlement given to a specimen provider as a compensation for providing a specimen; and a process that associates genome information obtained through an analysis of the provided specimen and identification information of the specimen provider with the generated information that can be exchanged for value and stores the associated information in a database

## Description

### Technical Field

This disclosure relates to a server device and a service providing system that provide genome related services.

### Background Art

Recently, development of genomic analysis technique has been advancing and utilization of genome information has been popular. For example, when a doctor gives an anticancer drug treatment to a patient, DNA (specimen) of the patient is preliminarily collected and sent to an inspection center. In the inspection center, for example, an inspection is performed using a genetic test system like a DNA chip to identify its DNA type. Finding the DNA type allows preliminarily determining presence/absence of a side effect of an administered medicine, presence/absence of efficacy of the medicine, and the like. This eliminates the need for trial administration that had been previously performed, making it possible to administer a medicine having efficacy and no (small) side effect to this patient from the beginning. Therefore, further utilization of the DNA information (genome information) has been recently desired.

In this respect, an object of Patent Literature 1 is "to provide an integrated database system that incorporates genome information into a clinical information database contrary to the conventional databases, while complying with the guidelines preventing identification of a provider of the genome information, thereby enabling an easy search for a correlation between the genome information and clinical information and the like", and Patent Literature 1 discloses the integrated database system of the genome information and the clinical information that "includes an information database including a data storage unit for storing clinical information of a plurality of patients and genome information of at least a part of the plurality of patients. In the data storage unit, the clinical information does not include personal information that identifies the individual patients, and the genome information and the clinical information of the same patient are stored in association with each other by link information that does not identify the individual patients."

### Citation List

### Patent Literature

Patent Literature 1: JP 2009-70096 A

### Summary of Invention

### Technical Problem

However, in Patent Literature 1, the database of only the genome information that has been already locally present is made, and Patent Literature 1 lacks in a viewpoint that the genome information is further extensively collected from persons (referred to as healthy persons) other than persons (patients) who have been confirmed to be currently affected with some sort of illness and the like for utilization. It is considered that when the genome information can be extensively collected from the persons other than the patients, the genome information can be considerably effectively utilized.

Generally, although it is expected that persons who are willing to provide the genome information (specimens) by a spirit of service are present, it has to be stated that its proportion is small. Accordingly, establishing a mechanism by which general persons are motivated to provide the genome information (specimens) is considerably important.

This disclosure has been made in consideration of such a situation and provides a technique that allows extensively collecting genome information (specimens) from general persons other than patients, and allows effective utilization of the genome information.

### Solution to Problem

In order to solve the problem, a server device according to this disclosure provides genome related services. The server device includes a storage device and a processor. The storage device stores a program to provide the service. The processor reads the program from the storage device and executes the program. The processor executes: a process that generates information that can be exchanged for value (value exchange information) for predetermined settlement given to a specimen provider as a compensation for providing a specimen; and a process that associates genome information obtained through an analysis of the provided specimen and identification information of the specimen provider with the generated information that can be exchanged for value and stores the associated information in a database.

A service providing system according to this disclosure includes a server device, a first computer of a specimen provider, and a second computer of a utilization requester. The server device provides genome related services. The first computer of the specimen provider provides a specimen to collect genome information. The second computer is of the utilization requester who desires to utilize the genome information. The server device, the first computer, and the second computer are connected over a network. (i) The server device executes: a process that generates information that can be exchanged for value for predetermined settlement given to the specimen provider as a compensation for providing the specimen; a process that associates the genome information obtained through an analysis of the provided specimen and identification information of the specimen provider with the generated information that can be exchanged for value and stores the associated information in a database; a process that acquires the genome information of the specimen provider and the given information that can be exchanged for value from the database in response to a browsing request of the genome information of the specimen provider received from the first computer and allows the specimen provider to browse the acquired information; a process that receives a genome information acquisition request including a desired condition of desired genome information received from the second computer; a process that searches the database based on the desired condition included in the genome information acquisition request and identifies the specimen provider matching the condition; and a process that transmits the genome information of the specimen provider matching the desired condition to the second computer or a process that permits an access to the genome information of the specimen provider matching the desired condition from the second computer. (ii) The first computer executes: a process that transmits the browsing request to the server device; and a process that displays the genome information of the specimen provider and the information that can be exchanged for value on a display screen of the first computer. (iii) The second computer executes: a process that transmits the genome information acquisition request to the server device; and a process that acquires the genome information of the specimen provider matching the condition from the server device, or a process that accesses the server device to access the genome information of the specimen provider matching the condition.

Further features related to this disclosure are clarified from the descriptions of this specification and the accompanying drawings. The aspects of this disclosure can be accomplished and achieved by components and combinations of various components, and the following detailed description and the aspects of the accompanying claims. It should be understood that the descriptions of this specification are merely typical examples and therefore do not limit the claims or application examples by any means.

### Advantageous Effects of Invention

This disclosure allows extensively collecting genome information (specimens) from general persons other than patients and allows effective utilization of the genome information.

### Brief Description of Drawings

Fig. 1 is a drawing illustrating an exemplary overall configuration of a service providing system 1 that manages genome information and executes a service related to the genome information according to this embodiment.
Fig. 2 is a drawing illustrating an exemplary internal configuration of terminal devices 10_1 to 10_n of specimen providers 1 to ***n.***
Fig. 3 is a drawing illustrating an exemplary internal configuration of a management server 20.
Fig. 4 is a drawing illustrating an exemplary internal configuration of a management server 30 of a DB management company.
Fig. 5 is a drawing illustrating an exemplary internal configuration of terminal devices 40_1 to 40_n of clients (companies: such as pharmaceutical companies) 1 to ***n*.**
Fig. 6 is a drawing illustrating an exemplary internal configuration of terminal devices 50_1 to 50_n of medical institutions or the like (such as hospitals) 1 to ***n.***
Fig. 7 is a drawing illustrating an overall configuration of a genome information database 100.
Fig. 8 is a drawing illustrating detailed contents of genome information 1003 as constitution items of the genome information database 100.
Fig. 9 is a drawing illustrating detailed contents of identification information 1004 as constitution items of the genome information database 100.
Fig. 10 is a drawing illustrating detailed contents of token information 1005 as constitution items of the genome information database 100.
Fig. 11 is a drawing illustrating detailed contents of genome information utilization history information 106 as constitution items of the genome information database 100.
Fig. 12 is a flowchart for describing a registration process of the genome information and the identification information in the genome information database 100 and a comprehensive agreement confirmation process executed between a terminal device 10_k (***k*** = 1 to ***n***) of the specimen provider and the management server 20 of a service providing company.
Fig. 13 is a flowchart for describing a browsing request process of these registered genome information and identification information of the specimen provider himself/herself executed between the terminal device 10_k (***k*** = 1 to ***n***) of the specimen provider and the management server 20 of the service providing company.
Fig. 14 is a flowchart for describing of a process that processes a genome information acquisition/browsing request from a terminal device 40_k (***k*** = 1 to ***n***) of a client company and discloses the desired genome information.
Fig. 15 is a flowchart for describing a process to use a token given to the specimen provider.
Fig. 16 is a flowchart for describing a registration process of a specimen with clinical information (which includes the genome information when specimen analysis has been performed and the genome information is present) stored in a biobank by a medical institution or the like in the genome information database 100.
Fig. 17 is a flowchart for describing a process when the medical institution or the like transmits a browsing request of the specimen with clinical information and the genome information registered in the genome information database 100 to the management server 20 of the service providing company.
Fig. 18 is a flowchart for describing a sequence of processes when the client company (such as a pharmaceutical company) acquires the desired genome information from the service providing company and makes a clinical trial collaboration request to the target medical institution or the like.

### Description of Embodiments

The following describes embodiments and respective examples of this disclosure with reference to the accompanying drawings. The accompanying drawings represent functionally the same components by the same reference numerals in some cases. Although the accompanying drawings illustrate the specific embodiments and implementation examples according to a principle of this disclosure, these drawings are for understanding of this disclosure and never used for limited interpretation of this disclosure.

While the embodiments give the description in detail enough for a person skilled in the art to carry out this disclosure, it is necessary to understand that other implementations and configurations are possible and changes in configurations and structures and substitutions of various components can be made without departing from the scope or the spirit of the technical idea of this disclosure. Therefore, the following description should not be interpreted to be limited.

### <Exemplary Overall Configuration of System>

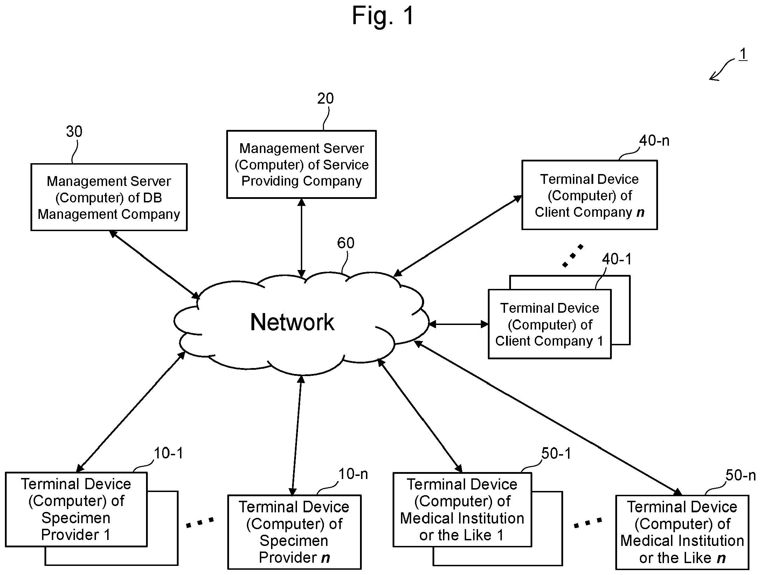

Fig. 1 is a drawing illustrating an exemplary overall configuration of a service providing system 1 that manages genome information and executes a service related to the genome information according to this embodiment.

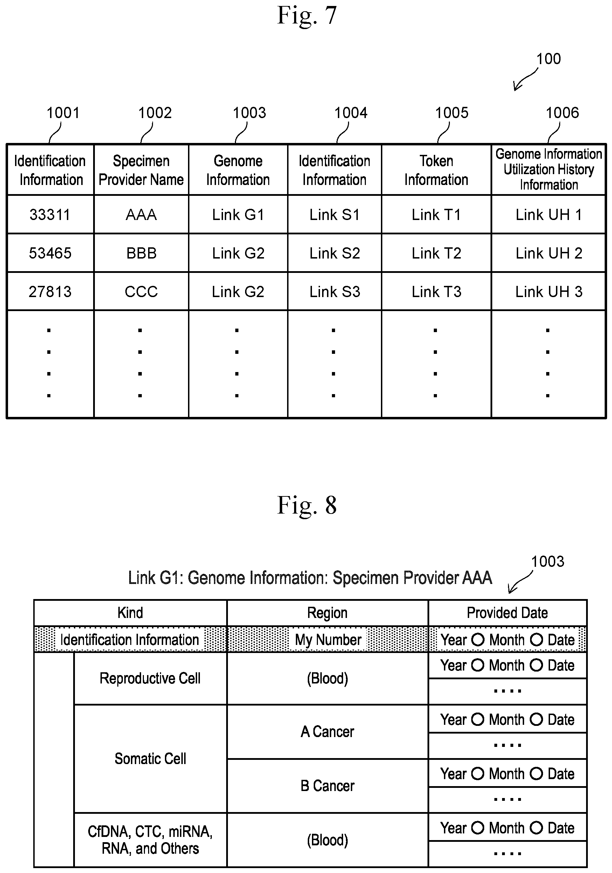

The service providing system 1 includes, for example, terminal devices (mobile terminals and fixed terminals) 10_1 to 10_n of specimen providers, a management server 20 of a service providing company, a management server 30 of a Database (DB) management company, terminal devices 40_1 to 40_n of client companies 1 to ***n*,** and terminal devices 50_1 to 50_n of medical institutions or the like (for example, hospitals) 1 to ***n*.** The terminal devices 10_1 to 10_n are held or possessed by respective specimen providers 1 to ***n*** to be operated and access a network. The management server 20 manages information and data transmitted from the terminal devices 10_1 to 10_n of the specimen providers 1 to ***n*** and information and data transmitted from a terminal device of a client company, gives and manages tokens (for example, information that can be exchanged for value distributed in a predetermined community as a compensation for provision of a specimen and disclosure of genome information) as a compensation for providing specimens by the specimen providers, and further provides a service, such as disclosure of the genome information to each client company. The management server 30 manages genome information databases (details will be described later: see from Fig. 7 to Fig. 11) (note that the management server 30 of the DB management company may be integrated with the management server 20 of the service providing company (may be the same server)). The terminal devices 40_1 to 40_n are permitted to browse the desired genome information from the management server 20 of the service providing company or receive its provision on the condition of purchase of the token. These devices are connected over a network 60 so as to ensure mutual communications.

### <Exemplary Internal Component of Each Component in System>

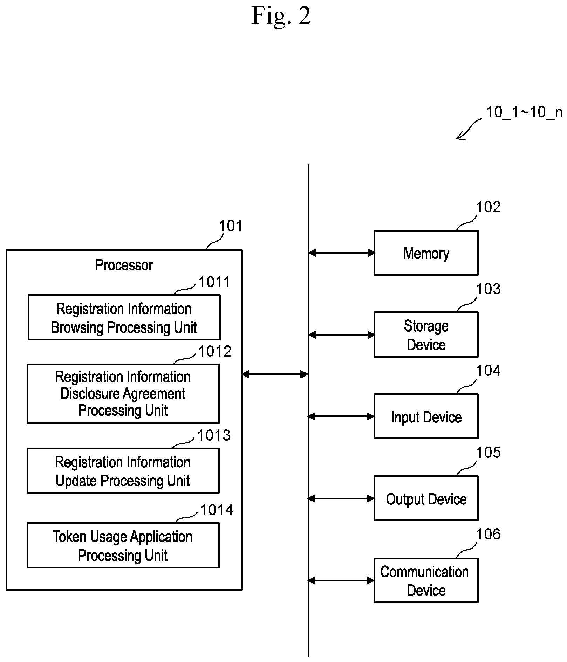

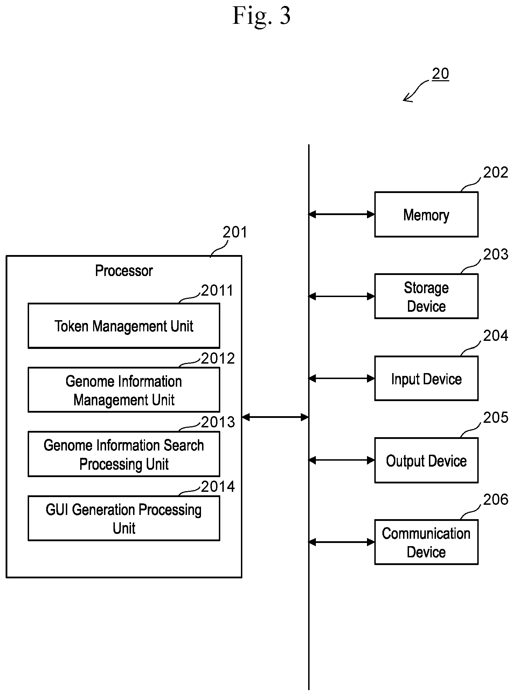

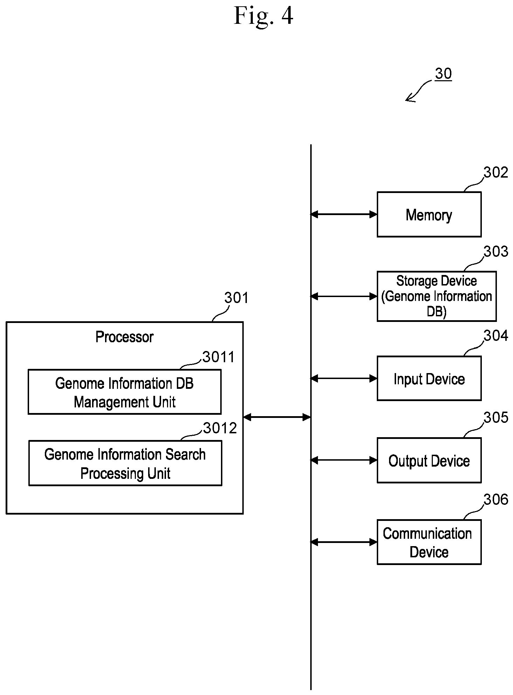

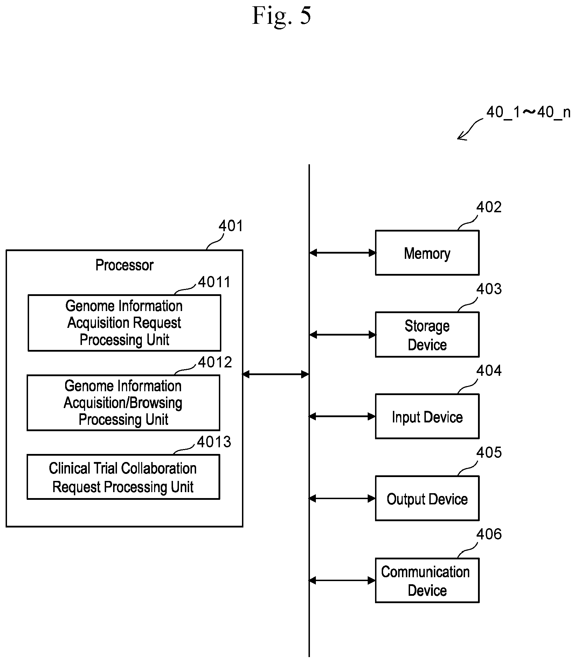

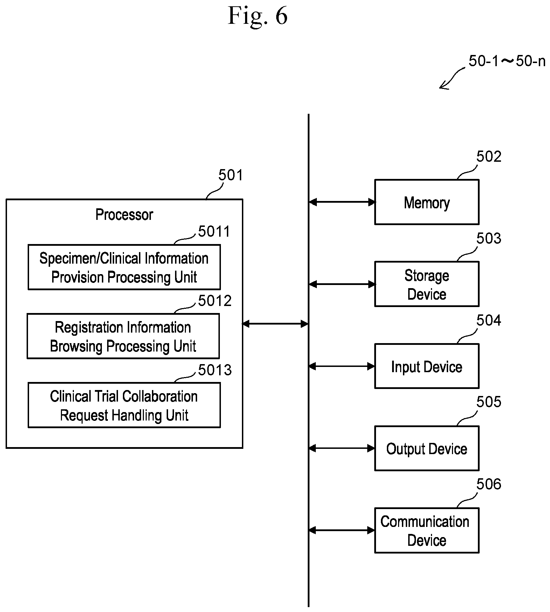

Fig. 2 to Fig. 6 are drawings illustrating an exemplary internal configuration of each device constituting the service providing system 1. Fig. 2 is a drawing illustrating an exemplary internal configuration of the terminal devices 10_1 to 10_n of the specimen providers 1 to ***n*.** Fig. 3 is a drawing illustrating an exemplary internal configuration of the management server 20. Fig. 4 is a drawing illustrating an exemplary internal configuration of the management server 30 of the DB management company. Fig. 5 is a drawing illustrating an exemplary internal configuration of the terminal devices 40_1 to 40_n of the clients (companies: such as pharmaceutical companies) 1 to ***n*.** Fig. 6 is a drawing illustrating an exemplary internal configuration of the terminal devices 50_1 to 50_n of the medical institutions or the like (such as hospitals) 1 to ***n*.** As illustrated in Fig. 2 to Fig. 6, the respective devices constituting the service providing system 1 are configured by computers.

### (i) Terminal Device of Specimen Provider

As illustrated in Fig. 2, the terminal devices 10_1 to 10_n of the specimen providers include processors 101, memories 102, storage devices 103, input devices 104, output devices 105, and communication devices 106. The processor 101 executes various programs installed on the terminal device. The memory 102 stores the various programs and various parameters. The storage device 103 stores calculation results and acquired information. The input device 104 includes a keyboard, a touchscreen, various buttons, a microphone, and the like (may be one of these devices or may include a plurality of types of the input devices). The output device 105 includes a display device, a printer, a speaker, and the like (may be one of these devices or may include a plurality of types of the output devices). The communication device 106 connects to the network 60 for communications with external devices, such as the management server 20 of the service providing company, the management server 30 of the DB management company, the terminal devices 40_1 to 40_n of the client companies 1 to ***n*,** and the terminal devices 50_1 to 50_n of the medical institutions or the like 1 to ***n*.** Although not illustrated, for example, when the terminal devices 10_1 to 10_n are mobile terminals, the terminal devices 10_1 to 10_n may include GPS functions, acceleration sensors, vibration sensors, pulse sensors, direction sensors, gyro sensors, and the like. These sensors may detect the current health condition and frequency of exercise of the specimen provider and the like, transmit sensed information to the management server 20, and register the information as additional information of the genome information database.

The processor 101 reads various programs from the memory 102, develops these programs into a built-in memory (not illustrated), and executes the programs as necessary. Fig. 2 illustrates a state in which the various programs are developed into a built-in memory of the processor 101. The processor 101 executes a registration information browsing processing unit 1011, a registration information disclosure agreement processing unit 1012, a registration information update processing unit 1013, and a token usage application processing unit 1014 as the programs.

For example, the registration information browsing processing unit 1011 transmits an application for browsing the genome information of the specimen provider himself/herself, which is the genome information corresponding to the specimen sent (provided) to the service providing company by the specimen provider, registered in the genome information database managed in the management server 20 or 30 by the service providing company or the DB management company to the management server 20 of the service providing company. The registration information browsing processing unit 1011 receives browsing Graphical User Interface (GUI) data generated by the management server 20 in response to the transmitted browsing application, re-constructs this browsing GUI data, and displays the browsing GUI data on a screen of the output device (display device) 105. The browsing GUI data includes, for example, any registration information including the genome information of the specimen provider himself/herself, the amount (quantity) of the given token, and personal information.

The registration information disclosure agreement processing unit 1012 processes a response to an inquiry about the disclosure of the genome information of the specimen provider. For example, when the management server 20 of the service providing company registers the genome information obtained from the specimen of the specimen provider in the genome information database, the management server 20 inquiries whether to comprehensively agree (permit) to the disclosure of this genome information to a third person or to desire to receive an inquiry of agreement (permission) on each future disclosure permission request to individually determine to agree or not. In response to this inquiry, when the specimen provider desires the comprehensive agreement, the registration information disclosure agreement processing unit 1012 transmits information indicative of declaration of intention of the comprehensive agreement to the management server 20 in accordance with the input instruction for notifying the intention by the specimen provider. When the specimen provider does not desire the comprehensive agreement but desires to determine whether to make an agreement individually corresponding to the future disclosure request, the registration information disclosure agreement processing unit 1012 transmits the information indicative of the declaration of intention of individual agreement to the management server 20 in accordance with the input instruction for notifying the intention by the specimen provider. For example, a declaration of intention form that agrees to disclosure under a predetermined condition (such as a category of business of a company to be disclosed, a disclose timing, and a nationality of the company to be disclosed), such as although disclosure to the pharmaceutical companies is agreed, disclosure to companies other than pharmaceutical companies being not permitted, may be employed.

When the specimen provider desires to update the information previously registered, the registration information update processing unit 1013 is started and new information of an updated item is input using the input device 104. In response to the information input and the information transmission instruction by the specimen provider, the registration information update processing unit 1013 transmits the input update information (including the information of the updated item) to the management server 20.

When the specimen provider desires to be diagnosed and/or receive treatment in the medical institution or the like (such as a registered hospital in the token distributed community), the specimen provider applies for the usage of the token to the service providing company. In this case, the specimen provider starts the token usage application processing unit 1014 and inputs the information on the medical institution or the like where the specimen provider declares the intention of the usage of token, is diagnosed, or is treated using the input device 104. In response to the information input and the information transmission instruction by the specimen provider, the token usage application processing unit 1014 transmits the declaration of intention of the usage of token and the information of the selected medical institution or the like to the management server 20.

### (ii) Management Server of Service Providing Company

The management server 20 is a server computer of the service providing company that gives the token as the compensation for the provision of the specimen to the specimen provider and manages the genome information of the specimen provider together with the information of the amount of this given token and the personal information of the specimen provider. Additionally, the management server 20 permits the acquisition or browsing of the genome information matching a request condition to a desired genome information acquisition/browsing request from the client company on the assumption of the purchase of the token and the agreement by the specimen provider.

As illustrated in Fig. 3, the management server 20 includes a processor 201, a memory 202, a storage device 203, an input device 204, an output device 205, and a communication device 206. The processor 201 executes installed various programs. The memory 202 stores the various programs and various parameters. The storage device 203 stores calculation results, acquired information, and the genome information database. The input device 204 includes a keyboard, a touchscreen, various buttons, a microphone, and the like (may be one of these devices or may include a plurality of types of the input devices). The output device 205 includes a display device, a printer, a speaker, and the like (may be one of these devices or may include a plurality of types of the output devices). The communication device 206 connects to the network 60 for communications with external the devices, such as the terminal devices 10_1 to 10_n of the specimen providers, the management server 30 of the DB management company, the terminal devices 40_1 to 40_n of the client companies 1 to ***n*,** and the terminal devices 50_1 to 50_n of the medical institutions or the like 1 to ***n.*** Although the description has been given that the genome information database is provided in the management server 20 of the service providing company here, the genome information database may be provided in the management server 30 of the DB management company or may be provided in both of the management server 20 and the management server 30. Alternatively, the whole genome information databases may be provided in the management server 30 of the DB management company and some of the genome information databases may be stored in the management server 20.

The processor 201 reads various programs from the memory 202, develops these programs into a built-in memory (not illustrated), and executes the programs as necessary. Fig. 3 illustrates a state in which the various programs are developed into a built-in memory of the processor 201. The processor 201 executes a token management unit 2011, a genome information management unit 2012, a genome information search processing unit 2013, and a GUI generation processing unit 2014 as the programs.

For example, the token management unit 2011 issues the token as the compensation for the provision of the specimen to the specimen provider and gives the token. Further, when the specimen provider discloses further detailed information, the token management unit 2011 issues and gives an additional token. The token management unit 2011 associates the information of the given token with identification information (ID) of the specimen provider and stores the information in the genome information database. When the management server 30 of the DB management company manages all or some of the genome information, the personal information, and the token information of the specimen provider, the token management unit 2011 transmits the given token information to the management server 30 of the DB management company using the communication device 206.

After the specimen is provided from the specimen provider and the genome information obtained from this specimen is acquired, the genome information management unit 2012 associates the information with the identification information of this specimen provider and stores the genome information obtained from the specimen to the genome information database

(storage device 203). When the management server 30 of the DB management company manages the genome information database, the genome information management unit 2012 transmits the genome information associated with the identification information of the specimen provider to the management server 30 of the DB management company via the communication device 206. Additionally, the genome information management unit 2012 receives the detailed personal information (identification information) input by the specimen providers 1 to ***n*** via a terminal device 10_k (***k*** = an integer from 1 to ***n***) and transmitted over the network, associates the information with the identification information of the target specimen provider, and stores the information in the genome information database (storage device 203) (or transmits the information to the management server 30 of the DB management company).

When the genome information management unit 2012 receives the browsing request of the information (the genome information and the identification information) registered by the specimen provider himself/herself from the terminal device 10_k (***k*** = an integer any of from 1 to ***n***) of the specimen provider, the genome information management unit 2012 acquires the information corresponding to the browsing request from the genome information database. Then, the genome information management unit 2012 instructs the GUI generation processing unit 2014 to generate the browsing GUI. The GUI generation processing unit 2014 generates the browsing GUI data to display the information acquired from the genome information database and transmits this browsing GUI data to the terminal device 10_k of the specimen provider via the communication device 206.

The genome information search processing unit 2013 receives a genome information search request including a desired search condition from, for example, any of a terminal devices 40_k (***k*** = an integer from 1 to ***n***) of the client companies ***k*** and acquires the genome information matching the search condition from the genome information database. The genome information search processing unit 2013 further classifies the search result (the genome information (including the identification information of the specimen provider)) in accordance with the presence/absence of disclosure agreement by each specimen provider and allows the terminal device 40_k of the client company ***k*** that has transmitted the genome information search request to acquire or browse only the genome information whose disclosure has been agreed. When the terminal device 40_k of the target client company ***k*** is permitted to only browse the genome information (including the identification information), the GUI generation processing unit 2014 may generate the browsing GUI data (data that cannot be modified) and transmit the data to the terminal device 40_k of the client company ***k,*** or the terminal device 40_k of the client company ***k*** may be accessible to the genome information matching the search condition and whose disclosure has been agreed temporarily stored in the memory 202 (one example) in the management server 20 of the service providing company.

### (iii) Server of DB Management Company

The management server 30 of the DB management company is a computer that receives the genome information and the detailed personal information (identification information) of each specimen provider from the management server 20 of the service providing company, stores the information in the genome information database, and manages the genome information, the identification information, and the information of the given token of each specimen provider. Note that when all pieces of the information in this genome information database are managed by the management server 20 of the service providing company, disposing the management server 30 of the DB management company is unnecessary. When the genome information database is provided in the management server 30 of the DB management company, the management server 30 of the DB management company may execute the process of the genome information search request from the terminal device 40_k of the client company ***k*.**

As illustrated in Fig. 4, the management server 30 of the DB management company includes a processor 301, a memory 302, a storage device 303, an input device 304, an output device 305, and a communication device 306. The processor 301 executes installed various programs. The memory 302 stores the various programs and various parameters. The storage device 303 stores calculation results, acquired information, and the genome information database. The input device 304 includes a keyboard, a touchscreen, various buttons, a microphone, and the like (may be one of these devices or may include a plurality of types of the input devices). The output device 305 includes a display device, a printer, a speaker, and the like (may be one of these devices or may include a plurality of types of the output devices). The communication device 306 connects to the network 60 for communications with the external devices, such as the terminal devices 10_1 to 10_n of the specimen providers, the management server 20 of the service providing company, the terminal devices 40_1 to 40_n of the client companies 1 to ***n*,** and the terminal devices 50_1 to 50_n of the medical institutions or the like 1 to ***n*.**

The processor 301 reads various programs from the memory 302, develops these programs into a built-in memory (not illustrated), and executes the programs as necessary. Fig. 4 illustrates a state in which the various programs are developed into a built-in memory of the processor 301. The processor 301 executes a genome information DB management unit 3011 and a genome information search processing unit 3012 as the programs.

The genome information DB management unit 3011 operates similarly to the above-described genome information management unit 2012 in the management server 20. For example, the genome information DB management unit 3011 receives the browsing request transmitted from the terminal device 10_k (***k*** = an integer from 1 to n) of the specimen provider ***k*** via the management server 20 of the service providing company, acquires the genome information and the identification information of the specimen provider ***k*** from the genome information database, and replies to the management server 20 of the service providing company. When the information is received, the management server 20 generates the browsing GUI data using the GUI generation processing unit 2014 and transmits the browsing GUI data to the terminal device 10_k of the specimen provider ***k*.** Note that when the management server 30 of the DB management company has a function corresponding to the GUI generation processing unit 2014, the management server 30 of the DB management company may directly transmit the browsing GUI data to the terminal device 10_k of the specimen provider ***k*** over the network 60.

The genome information search processing unit 3012 operates similarly to the above-described genome information search processing unit 2013 in the management server 20. For example, the genome information search processing unit 3012 receives the genome information search request including the desired search condition transmitted from the terminal device 40_k (***k*** = an integer from 1 to ***n***) of the client company ***k*** via the management server 20 of the service providing company and acquires the genome information (including the identification information) matching the search condition from the genome information database. Then, the genome information search processing unit 3012 transmits the acquired genome information (including the identification information) to the management server 20 of the service providing company. The management server 20 of the service providing company that has received the search result (the genome information and the identification information) from the management server 30 of the DB management company, as described above, classifies the search result in accordance with the presence/absence of disclosure agreement by each specimen provider and allows the terminal device 40_k of the client company ***k*** that has transmitted the genome information search request to acquire or browse only the genome information whose disclosure has been agreed.

### (iv) Terminal Device of Client Company

The terminal devices 40_1 to 40_n of the client companies 1 to ***n*** are computers that transmit the genome information search request including the desired search condition to the management server 20 of the service providing company and acquires or browses the genome information (including the identification information) of the specimen provider as the search result corresponding to this request and whose disclosure has been agreed. In a case where the client companies 1 to ***n*** are pharmaceutical companies, the terminal devices 40_1 to 40_n of the client companies (pharmaceutical companies) 1 to ***n*** transmit a clinical trial collaboration request to the terminal device 50_k (***k*** = 1 to ***n***) of the medical institution or the like and receive a response to the request. Note that, to receive these services (access to the genome information and the clinical trial collaboration request), the client companies 1 to ***n*** need to purchase the above-described tokens by a predetermined amount.

As illustrated in Fig. 5, the terminal devices 40_1 to 40_n of the client companies 1 to ***n*** include processors 401, memories 402, storage devices 403, input devices 404, output devices 405, and communication devices 406. The processor 401 executes installed various programs. The memory 402 stores the various programs and various parameters. The storage device 403 stores calculation results and acquired information. The input device 404 includes a keyboard, a touchscreen, various buttons, a microphone, and the like (may be one of these devices or may include a plurality of types of the input devices). The output device 405 includes a display device, a printer, a speaker, and the like (may be one of these devices or may include a plurality of types of the output devices). The communication device 406 connects to the network 60 for communications with the external devices, such as the terminal devices 10_1 to 10_n of the specimen providers, the management server 20 of the service providing company, the management server 30 of the DB management company, and the terminal devices 50_1 to 50_n of the medical institutions or the like 1 to ***n.***

The processor 401 reads various programs from the memory 402, develops these programs into a built-in memory (not illustrated), and executes the programs as necessary. Fig. 5 illustrates a state in which the various programs are developed into a built-in memory of the processor 401. The processor 401 executes a genome information acquisition request processing unit 4011 and a genome information acquisition/browsing processing unit 4012 as the programs. When the client companies 1 to ***n*** are pharmaceutical companies, the processor 401 further executes a clinical trial collaboration request processing unit 4013.

The genome information acquisition request processing unit 4011 acquires the desired search condition input using the input device 404 by a person in charge of the client company, converts the desired search condition into a predetermined data format for searching, and transmits it to the management server 20 of the service providing company via the communication device 406. The input search condition is a feature of the genome information desired to be acquired or browsed. The input search condition includes at least one item selected from, for example, a sex, a height, a weight, an age (era), presence/absence of current affection of specific disease, a past medical history, previous illnesses, past injuries, presence/absence of experience of blood transfusion, presence/absence of specific allergy, lifestyle habits (drinking habits and smoking habits), case histories of family and a relative of the specimen provider, and the like. For example, in a case where the client company is a pharmaceutical company and desires to access the target genome information to discover a drug, the input search condition includes a specimen provider classified into a control and a specimen provider affected with a specific disease (a target disease to discover the drug).

The genome information acquisition/browsing processing unit 4012 acquires the genome information (including the identification information) as the search result transmitted from the management server 20 of the service providing company or the browsing GUI data of the genome information and displays it in a display screen of the output device (example: display device) (since the browsing GUI data is transmitted as, for example, HTML or XML data, the data is reconstructed and is displayed using software for browsing). In the configuration in which the information of browning permission is received from the management server 20 of the service providing company, the genome information acquisition/browsing processing unit 4012 accesses (on the condition of inputting security information, such as a password included in the information of browning permission) browsing data provided in the management server 20 of the service providing company using the communication device 406, acquires the desired genome information (identification information) or the browsing data, and displays it on the screen of the output device.

When the pharmaceutical company (client company) ***k*** (***k*** = 1 to ***n***) desires to purchase the token from the service providing company and receive the clinical trial collaboration from any of the medical institutions or the like 1 to ***n*,** the clinical trial collaboration request processing unit 4013 transmits clinical information (information indicating that clinical trial is desired to be performed to what sort of patient (condition for clinical trial)) input by the person in charge of the pharmaceutical company using the input device to the management server 20 of the service providing company. The management server 20 of the service providing company receives the clinical information, identifies the medical institution or the like (the hospital participating in the above-described token community) ***k*** that diagnoses and/or treats a patient matching the condition for clinical trial and declares an intention of clinical trial collaboration, notifies the terminal device 40_k of the client company (pharmaceutical company) ***k*** of the information of the medical institution or the like ***k*** (***k*** = 1 to ***n**;* a plurality of the medical institutions or the like may be identified, or the upper limit of the number of medical institutions or the like notified may be determined according to the amount of purchased token), and permits browsing the genome information and the identification information of the specimen provider matching the condition for clinical trial (any one or both of the information transmission to the terminal device 40_k and the access from the terminal device 40_k). In response to the reception of the information of the identified medical institution or the like ***k,*** the clinical trial collaboration request processing unit 4013 transmits the clinical trial collaboration request to the terminal device 50_k of this medical institution or the like ***k*** by the input instruction by the person in charge of the pharmaceutical company or by automatically, and receives information whether to perform the clinical trial collaboration from the terminal device 50_k of this medical institution or the like ***k.***

### (v) Terminal Device of Medical Institution or the Like

The terminal devices 50_1 to 50_n of the medical institutions or the like 1 to ***n*** are computers that execute a process related to the provision of the specimen with clinical information and/or the genome information stored in a biobank managed by each medical institution or the like and handle the clinical trial collaboration request from the pharmaceutical company. Note that providing various kinds of information stored in the biobank from each of the medical institutions or the like 1 to ***n*** to the service providing company allows receiving the token. These tokens are managed in the genome information database made correspond to the identification information of the medical institutions or the like 1 to ***n*.**

As illustrated in Fig. 6, the terminal devices 50_1 to 50_n of the medical institutions or the like 1 to ***n*** include processors 501, memories 502, storage devices 503, input devices 504, output devices 505, and communication devices 506. The processor 501 executes installed various programs. The memory 502 stores the various programs and various parameters. The storage device 503 stores calculation results and acquired information. The input device 504 includes a keyboard, a touchscreen, various buttons, a microphone, and the like (may be one of these devices or may include a plurality of types of the input devices). The output device 505 includes a display device, a printer, a speaker, and the like (may be one of these devices or may include a plurality of types of the output devices). The communication device 506 connects to the network 60 for communications with the external devices, such as the terminal devices 10_1 to 10_n of the specimen providers, the management server 20 of the service providing company, the management server 30 of the DB management company, and the terminal devices 40_1 to 40_n of the client companies 1 to ***n*.**

The processor 501 reads various programs from the memory 502, develops these programs into a built-in memory (not illustrated), and executes the programs as necessary. Fig. 6 illustrates a state in which the various programs are developed into a built-in memory of the processor 501. The processor 501 executes a specimen/clinical information provision processing unit 5011, a registration information browsing processing unit 5012, and a clinical trial collaboration request handling unit 5013 as the programs.

In a case where, for example, the medical institution or the like ***k*** (***k*** = 1 to ***n***) provides (for example, sends the specimen with clinical information using a home-delivery company) the specimen with clinical information to the service providing company, in response to the instruction by the person in charge of the medical institution or the like ***k,*** the specimen/clinical information provision processing unit 5011 notifies the management server 20 of the service providing company that this specimen has been sent. When a sending notification is received from the terminal device 50_k of the medical institution or the like ***k,*** after the reception of the specimen with clinical information is confirmed, the management server 20 of the service providing company responds to the instruction by the person in charge of the service providing company, links the genome information (the genome information is generated from the provided specimen) generated based on the provided specimen, the corresponding clinical information, and detailed information of the patient or the like related to the specimen (information, such as personal information and a case history), and the information of the amount of given token to the identification information of the medical institution or the like ***k,*** and registers the associated information in the genome information database. Then, the management server 20 of the service providing company notifies the terminal device 50_k of the medical institution or the like ***k*** of completion of the information registration to the genome information database (for example, may include an outline of the registered information) and the amount of token given as the compensation for provision of the specimen with clinical information. When the notification is received, the specimen/clinical information provision processing unit 5011 stores a registration date of the information corresponding to the provision of the specimen with clinical information to the genome information database, the registered outline, and the information on the amount of given token in the memory 502 or the storage device 503. Note that, when the person in charge of (the person involved in) the medical institution or the like ***k*** desires to update or delete the previously registered information, the person starts the specimen/clinical information provision processing unit 5011 and inputs new information of the updated item, a deletion request, or the like using the input device 504. Then, in response to the information input and the information transmission instruction by the person in charge (the person involved in) the medical institution or the like ***k,*** the specimen/clinical information provision processing unit 5011 transmits the input update information (including the information of the updated item), the deletion request, or the like to the management server 20.

For example, the registration information browsing processing unit 5012 transmits the application for browsing the information (the specimen/clinical information and the past and current detailed information of the patient related to the provided specimen provided by the medical institution or the like ***k***) registered in the genome information database managed in the management server 20 or 30 by the service providing company or the DB management company to the management server 20 of the service providing company. Then, in response to the transmitted browsing application, the registration information browsing processing unit 5012 receives the browsing Graphical User Interface (GUI) data generated by the management server 20, re-constructs this browsing GUI data, and displays the browsing GUI data on the screen of the output device (display device) 505. The browsing GUI data includes any registration information including the genome information based on the past/current specimen of the patient, the clinical information of this patient, the amount (quantity) of given token, the detailed information of the medical institution or the like ***k,*** and personal information and a case history of each patient provided by the medical institution or the like ***k.***

The clinical trial collaboration request handling unit 5013 receives the clinical trial collaboration request transmitted from the terminal device 40_k (***k*** = 1 to ***n***) of the client company (pharmaceutical company) ***k.*** The person in charge of (the person involved in) the medical institution or the like ***k*** examines the contents of the received clinical trial collaboration request and determines whether to perform the clinical trial collaboration. Then, the clinical trial collaboration request handling unit 5013 notifies the terminal device 40_k (***k*** = 1 to ***n***) of the client company (pharmaceutical company) ***k*** of whether to collaborate with the clinical trial. When the clinical trial is collaborated, the clinical trial collaboration request handling unit 5013 transmits a clinical trial participation request to the terminal device of each existing patient related to the specimen with clinical information provided to the service providing company. The terminal device of each existing patient can include a terminal device registered by the medical institution or the like ***k*** when the specimen is provided among the terminal devices 10_1 to 10_n of the specimen providers 1 to ***n*** and a terminal device other than the terminal devices 10_1 to 10_n.

### <Exemplary Configuration of Genome Information Database>

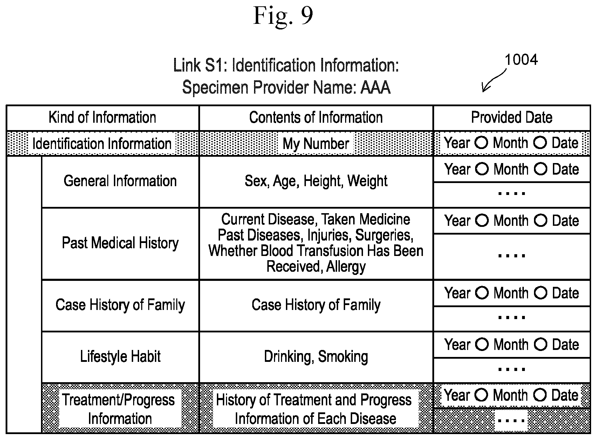

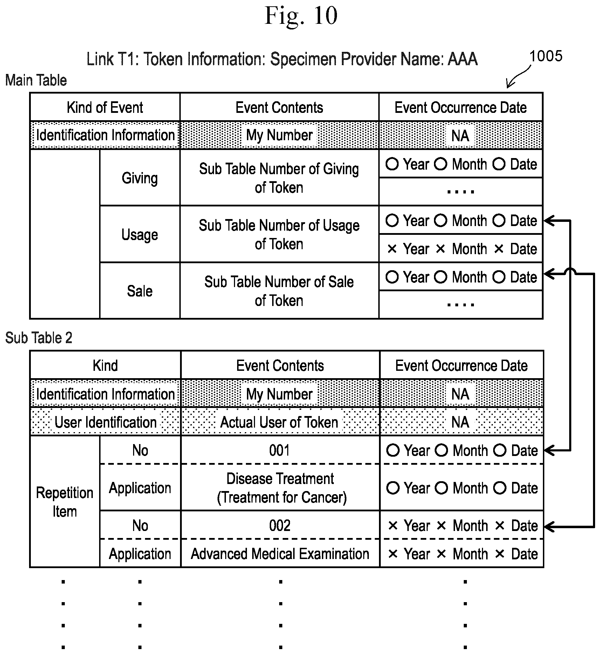

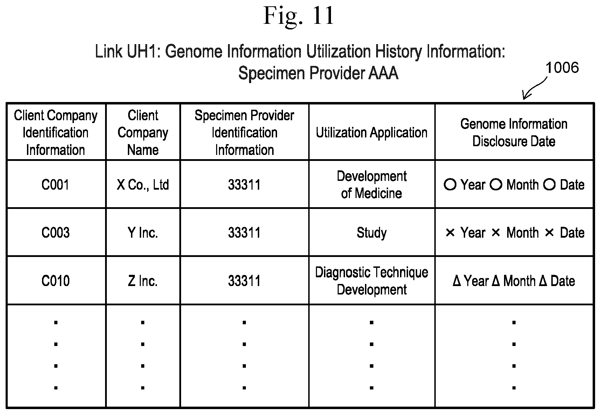

Fig. 7 to Fig. 11 are drawings illustrating exemplary configurations of the genome information database. Fig. 7 is a drawing illustrating the overall configuration of the genome information database 100. Fig. 8 is a drawing illustrating detailed contents of genome information 1003 as constitution items of the genome information database 100. Fig. 9 is a drawing illustrating detailed contents of identification information 1004 as constitution items of the genome information database 100. Fig. 10 is a drawing illustrating detailed contents of token information 1005 as constitution items of the genome information database 100. Fig. 11 is a drawing illustrating detailed contents of genome information utilization history information 1006 as constitution items of the genome information database 100. Although various pieces of information including the genome information database 100 and constituting the genome information database 100 are managed by table structure, this is merely an example, and as long as the various pieces of information are associated with one another and the information of each specimen provider can be acquired without an error, any structure may be used. The respective database components from Fig. 7 to Fig. 11 may be physically provided at one location or may be provided at different locations or as different databases. For example, a configuration in which the management server 20 includes the database components of Fig. 7, and the respective database components of from Fig. 8 to Fig. 11 are installed at different locations and are connected to the management server 20 over the network 60 may be used.

### (i) Overall Configuration of Genome Information Database (Fig. 7)

As illustrated in Fig. 7, the genome information database 100 includes identification information 1001, a specimen provider name 1002, the genome information 1003, the identification information 1004, the token information 1005, and the genome information utilization history information 1006 as the constitution items.

The identification information 1001 is information that uniquely identifies/discriminates the specimen provider. The service providing company may assign a unique number to each specimen provider, or the identification information 1001 may be a number issued by a public institution, such as a "Individual Number" (social security number), a health insurance card number, a driver's license number, and a passport number, the genome information, a unique character string generated based on the genome information, or the like. When the specimen is provided with a patient name remaining anonymous from the medical institution or the like, for example, a combination of the unique number identifying this institution and a number indicative of an order of provision of the specimens provided from this institution may be used as the identification information. Note that "Individual Number" is preferably used in the genome information database 100. This is because that "Individual Number" is a number unique to a person issued when the person was born. Therefore, the description of the genome information database 100 will be given using "Individual Number" in this embodiment.

The specimen provider name 1002 is the name of the specimen provider. As long as the identification information 1001 is present, the specimen provider name 1002 needs not to be the real name, and may be initials, a nickname, an anonymous, or the like.

The genome information 1003 manages information that the target specimen provider is what sort of specimen and the genome information acquired from the specimen. The detailed contents of the genome information 1003 may be directly described in (managed in) the genome information database 100 of Fig. 7, and as in this embodiment, the detailed contents may be described in (managed in) another table at a link destination (link G1). The details of the genome information 1003 will be described later (see Fig. 8).

The identification information 1004 manages information, such as general personal information, previous illnesses, and lifestyle habits of the target specimen provider. The detailed contents of the identification information 1004 may be directly described in (managed in) the genome information database 100 of Fig. 7 or may be described in (managed in) another table at a link destination (link S1) as in this embodiment. The details of the identification information 1004 will be described later (see Fig. 9).

The token information 1005 manages a token grant history (includes the amount of given token), a token usage history, a sold token history of the target specimen provider, and the like. The detailed contents of the token information 1005 may be described in (managed in) the genome information database 100 of Fig. 7 or may be described in (managed in) another table at a link destination

(link T1) as in this embodiment. The details of the token information 1005 will be described later (see Fig. 10).

The genome information utilization history information 1006 manages how the genome information of the target specimen provider is utilized by which client company. The detailed contents of the genome information utilization history information 1006 may be directly described in (managed in) the genome information database 100 of Fig. 7 or may be described in (managed in) another table at a link destination (link UH1) as in this embodiment. The details of the genome information utilization history information 1006 will be described later (see Fig. 11).

In the genome information database 100, the respective items of the above-described specimen provider name 1002 to the genome information utilization history information 1006 and their detailed contents (from Fig. 8 to Fig. 11) are all associated (linked) using the identification information ("Individual Number", the genome information, the unique character string generated based on the genome information, and the like) 1001 of the specimen provider.

### (ii) Detailed Configuration Example of Genome Information (Fig. 8)

In the genome information database 100 in this embodiment, the genome information 1003 is provided by the separately provided table (Fig. 8) associated to a link G. As illustrated in Fig. 7, for example, details of the genome information of a specimen provider "AAA" identified by the identification information "33311" are stored in the link destination indicated by the link G1. The genome information 1003 at least includes the information of the provided specimen and the genome information obtained through analysis of it.

With reference to Fig. 8, the genome information 1003 includes the specimen of the specimen provider or the type of the genome, a region of the specimen, and a date when the specimen is provided corresponding to the identification information of the target specimen provider ("Individual Number", the genome information, the unique character string generated based on the genome information, and the like). The kind of the specimen or the kind of the genome information is the kind of the provided specimen and the genome information obtained through the analysis of this specimen. For example, a reproductive cell and a somatic cell (specimen), CfDNA, CTC, miRNA, RNA, and others (genome information) correspond to this. The region of the specimen indicates that the provided specimen has been collected from which part of the body of the provided specimen. For example, it is seen that the reproductive cell is obtained from the blood of the specimen provider, the somatic cells are obtained from an A cancer and a B cancer, and CfDNA, CTC, miRNA, and RNA, and others (genome information) are obtained through analysis of the blood. Additionally, each of the provided dates indicate a date when the specimen provider provided the identification information ("Individual Number", the genome information, the unique character string generated based on the genome information, and the like), a date when various cells were provided as the specimens, and a date when the provided specimen was analyzed to acquire the genome information (such as CfDNA).

### (iii) Detailed Configuration Example of Identification Information (Fig. 9)

In the genome information database 100 in this embodiment, the identification information 1004 is provided by the separately provided table (Fig. 9) associated to a link S. As illustrated in Fig. 7, for example, details of the identification information of the specimen provider "AAA" identified by the identification information "33311" are stored in the link destination indicated by the link S1.

With reference to Fig. 9, the identification information 1004 includes the kind of the information of the specimen provider, the contents of the information, and the date when this information is provided corresponding to the identification information ("Individual Number", the genome information, the unique character string generated based on the genome information, and the like) of the target specimen provider. The kind of the information is any undisclosed information (includes information generally desired to be kept secret by the specimen provider) including personal information other than the specimen or the genome information of the specimen provider. For example, the information includes general information, past medical history information, case history information of family, information related to lifestyle habits, treatment/progress information of illness, proteomics information, and metabolomics information, and at least one of them corresponds to the identification information 1004. The contents of information indicate detailed contents of various pieces of information. The general information is, for example, the sex, the age, the height, and the weight of the specimen provider. The information of previous illnesses includes, for example, at least one of a disease with which the specimen provider is currently affected (such as a disease name, a symptom, and a level (serious or not)), presence/absence of hospitalization, a medicine taken (such as a name of medicine and a frequency of taking), a disease affected in the past (such as a disease name, a level (serious or not), presence/absence of hospitalization, a symptom, and a period of treatment until healing), an injury suffered in the past (a region and a level of the injury, presence/absence of hospitalization (including a hospitalization period), and a period of treatment until healing), presence/absence of experience of a surgery (including a time), presence/absence of experience of receiving blood transfusion (including a time), presence/absence and a kind of allergy, or the like. The information of the case history of family includes information of, for example, a case history (a disease name, a level (serious or not), a symptom, a period of treatment, and whether the person is currently alive) of a relative within the third degree, and the like. The information of lifestyle habits includes information of, for example, habits of drinking and smoking and a life rhythm (such as usual wake-up time and bedtime, times and the number of meals, and palatability of meals) of the specimen provider. The treatment/progress information includes, for example, a history of treatment of the disease with which the specimen provider is currently affected, progress information, and the like. The provided date each indicates a date when the specimen provider provides the identification information ("Individual Number", the genome information, the unique character string generated based on the genome information, and the like) and a date when various pieces of information are provided.

### (iv) Token Information (Fig. 10)

In the genome information database 100 in this embodiment, the token information 1005 is provided by the separately provided table (Fig. 10) associated to a link T. As illustrated in Fig. 7, for example, details of the token information of the specimen provider "AAA" identified by the identification information "33311" are stored in the link destination indicated by the link T1.

With reference to Fig. 10, the token information 1005 includes a plurality of kinds of tables (or a similar structure) of a main table and sub tables. The main table of the token information 1005 includes a kind of event (transaction) related to the token of the specimen provider, contents of the event (transaction), and a date when this event occurred corresponding to the identification information of the target specimen provider ("Individual Number", the genome information, the unique character string generated based on the genome information, and the like). The kind of event includes, for example, giving of the token to the specimen provider, the usage of the token by the specimen provider or the like, the sale of the token by the specimen provider, and the like. For example, the event contents are link information indicative of a sub table number of the giving of the token in the main table. The event occurrence date indicates the date when each event (transaction) occurred, and, for example, in a case where the event is the giving of the token, the date when each token grant event occurred is input.

The sub table is a table provided according to the kind of each event in the main table, and stores information of describing the contents of the event item (such as giving, usage, and sale) in further detail. Fig. 10 illustrates an example of the sub table (sub table 2) for the usage event of token.

The sub table 2 is a table that manages an actual user (user identification: may be used by the specimen provider himself/herself, or may be used by a person concerned with the specimen provider) corresponding to the identification information ("Individual Number", the genome information, the unique character string generated based on the genome information, and the like) of the specimen provider (a person who possesses the token), contents of a specific application corresponding to identification management information (001, 002, ..., and so on), and an event occurrence date (date of usage). The contents of the sub table 2 are associated with "Usage" as the item of the main table by the event occurrence date and the identification number of the specimen provider. While Fig. 10 illustrates only the sub table 2 with "Usage" as the example, sub tables for, for example, "Giving" and "Sale" also have the similar configurations. Note that, in the sub table for "Giving," for example, "name of the target person to whom the token is given" is described as "User Identification" and an action (for example, the disclosure of the identification information and the like) of the specimen provider that triggers the token grant is managed as "Repetition Item" of "Contents" corresponding to the identification management information (001, 002, ..., and so on). Additionally, in the sub table for "Sale," for example, "name of a person who has sold the token" is described as "User Identification" and contents of the token sale (transferred to whom) are managed as "Repetition Item" of "Contents" corresponding to the identification management information (001, 002, ..., and so on).

### (v) Genome Information Utilization History Information (Fig. 11)

In the genome information database 100 in this embodiment, the genome information utilization history information 1006 is provided by the separately provided table (Fig. 11) associated to a link UH. As illustrated in Fig. 7, for example, details of the genome information utilization history information of the specimen provider "AAA" identified by the identification information "33311" are stored in the link destination indicated by the link UH1.

With reference to Fig. 11, the genome information utilization history information 1006 includes identification information of the client company that utilizes the target genome information of the specimen provider, a client company name, the identification information of the specimen provider, a utilization application of the genome information, and a date when the target genome information is disclosed to the client company as constitution items. The client company (for example, a pharmaceutical company) is a company that made a contract with the service providing company to receive the provision of the service and purchased the token. The genome information can be disclosed to each client company on the assumption of the purchase of the token, and each client company can use the genome information for various applications and purposes. The client company name is a company name that utilizes the genome information. While an abbreviated name, a popular name, and the like are acceptable as the client company name, the client company name cannot be anonymous. The specimen provider identification information is the identification information ("Individual Number", the genome information, the unique character string generated based on the genome information, and the like) of the specimen provider who has provided the specimen corresponding to the utilized genome information, and in the example of Fig. 11, "Individual Number" of the specimen provider AAA and the like are described. Since the genome information utilization history information (table) associated with the link UH1 is a table to manage the utilization history of the genome information of the specimen provider AAA, the identification information of the specimen provider AAA is described in all of these columns. The utilization application indicates that the client company has utilized the target genome information for what application and purpose, and, for example, the application includes discovery of a drug, study, diagnostic technique development, and the like, but the application is not limited to these applications. The genome information disclosure date indicates a date when the genome information of the corresponding specimen provider (the specimen provider AAA in Fig. 11) was disclosed to the client company in response to the genome information acquisition/browsing request from this client company.

(vi) As described above, the respective pieces of the information in the genome information database 100, namely, the identification information 1001, the specimen provider name 1002, the genome information 1003, the identification information 1004, the token information 1005, and the genome information utilization history information 1006 are all associated with the identification information 1001 of the specimen provider. In view of this, the usage of the identification information 1001 of the specimen provider allows easily acquiring desired information.

When the specimen or the identification information is provided from the specimen provider, the genome information management unit 2012 may search the genome information database 100 using the identification information of this specimen provider ("Individual Number", the genome information, the unique character string generated based on the genome information, and the like) to determine whether the specimen or the identification information has been provided from this specimen provider in the past (first time point). When the specimen or the identification information has been provided from the specimen provider in the past, the genome information management unit 2012 may associate the genome information or the identification information in the past (first time point) with the genome information or the identification information in this time (second time point) and store the associated information in the genome information database 100.

### <Genome Information and the Like Registration Process>

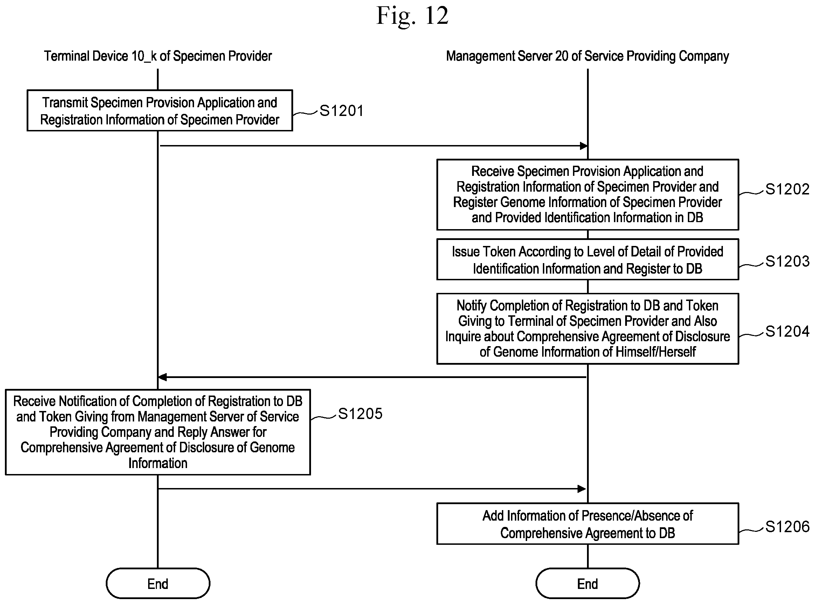

Fig. 12 is a flowchart for describing the registration process of the genome information and the identification information in the genome information database 100 and a comprehensive agreement confirmation process executed between the terminal device 10_k (***k*** = 1 to ***n***) of the specimen provider and the management server 20 of the service providing company. Note that Fig. 12 gives a description assuming that the genome information database 100 is provided in the management server 20 of the service providing company, but the genome information database 100 may be provided in the management server 30 of the DB management company. In the latter case, the management server 20 and the management server 30 communicate with one another to transmit or receive information. The genome information, obtained through the analysis of the specimen acquired from the specimen provider by the management server 20 of the service providing company, and the identification information are registered in the genome information database 100 provided in the management server 30 of the DB management company.

### (i) Step 1201

The specimen provider accesses (for example, accesses a service site of the service providing company) the management server 20 of the service providing company using the communication device 106 in the terminal device 10_k of the specimen provider and downloads a specimen provision application form or accesses a specimen provision application input screen. Then, using the input device 104, the specimen provider inputs the specimen provision application and the registration information of the specimen provider (for example, minimum necessary information constituting a part of the above-described identification information) in the specimen provision application form or directly inputs them to the specimen provision application input screen, and transmits it to the management server 20 of the service providing company using the communication device 106. At this time, the terminal device 10_k of the specimen provider receives a reception number (information with which the specimen by this specimen provider can be uniquely identified) from the management server 20 and stores the reception number in the memory 102 or the storage device 103.

Then, for example, the specimen provider goes to the medical institution or the like, the specimen of the specimen provider is collected, and the medical institution or the like delivers the specimen to the service providing company. Alternatively, after the specimen provider transmits the specimen provision application and receives the reception number, the specimen provider delivers the specimen of himself/herself to the service providing company together with this reception number.

### (ii) Step 1202

The management server 20 of the service providing company receives the specimen provision application and the registration information of the specimen provider from the terminal device 10_k of the specimen provider. In response to an instruction of the registration of the specimen provision application or the like input by the person in charge of the service providing company after confirmation of the reception of the specimen delivered from the target specimen provider or the medical institution or the like, the genome information management unit 2012 in the management server 20 of the service providing company registers the registration information of the specimen provider in the genome information database 100 as the above-described identification information. Since the genome information is obtained through the analysis of the specimen, the genome information is not registered in the genome information database 100 at this phase. However, in a case where the genome information is attached to the provided specimen (in a case where the specimen provider has already possessed a specimen analysis result (genome information) obtained through another institution), the genome information may be immediately registered in the genome information database 100, or the genome information may be temporarily registered, and after the specimen analysis by the service providing company is terminated, the temporarily registered genome information may be afterward switched to the genome information obtained through the analysis.

Note that, for example, the specimen provision application form can include entry columns regarding a plurality of items included in the above-described identification information. The specimen provider can optionally determine that information of which item is provided. Accordingly, a level of detail of the registration information differs depending on the specimen provider.

After the analysis of the specimen provided to the service providing company is terminated, the genome information management unit 2012 registers the analysis result in the genome information database 100 as the genome information.

### (iii) Step 1203

The token management unit 2011 in the management server 20 issues the token according to the level of detail of the provided registration information (identification information) and registers the amount of issued token in the token information 1005 in the genome information database 100. The amount of issued token is, for example, preliminarily set to each item of the identification information provided together with the specimen. The amount of token is set to be increased when information of the important item is provided and as the number of items increases. The token management unit 2011 compares the amount of token preliminarily set to each item of this identification information with the item of the provided registration information (identification information) to determine the amount of token to be issued.

### (iv) Step 1204

The genome information management unit 2012 notifies the terminal device 10_k of this specimen provider of completion of the registration of the genome information of the specimen provider and the provided identification information in the genome information database 100, the token grant, and completion of the registration in the genome information database 100 (token information 1005), and transmits an inquiry of presence/absence of an intention of comprehensive agreement regarding the disclosure of the genome information of himself/herself to the terminal device 10_***k**.*

### (v) Step 1205

(The processor 101 in) the terminal device 10_k of the specimen provider receives the notifications of the completion of registration to the database and the token grant from the management server 20 and also receives the inquiry regarding the comprehensive agreement to the genome information disclosure. This specimen provider determines whether to make the comprehensive agreement to the genome information disclosure and inputs own intention (whether to make the comprehensive agreement). In response to the instruction by the specimen provider, the registration information disclosure agreement processing unit 1012 replies an answer for the inquiry of the comprehensive agreement to the management server 20. When the comprehensive agreement is made, whether to perform disclosure will not be inquired afterward when the genome information of himself/herself is disclosed (access permission) to the client company. On the other hand, when the comprehensive agreement is not made, whenever the genome information of himself/herself is disclosed (access permission) to the client company, whether to perform disclosure is inquired in advance, and the specimen provider answers for the presence/absence of disclosure agreement individually.

### (vi) Step 1206

The genome information management unit 2012 in the management server 20 additionally registers the received information of the presence/absence of the intention of comprehensive agreement in the genome information database 100 (not illustrated in Fig. 7). In the case where the comprehensive agreement has been made, the token may be additionally issued as a compensation for it and the token may be added to the token information 1005 together with the event occurrence date (comprehensive agreement date). In the case where the individual agreement has been made as well, the token may be additionally issued as a compensation for it and the token may be added to the token information 1005 together with the event occurrence date (individual agreement date). The token of more amount may be issued in the case of comprehensive agreement. For example, a token equivalent to the amount of issued tokens at the time of the individual agreements by 10 times can be issued for comprehensive agreement.

### <Genome Information or the like Browsing Request Process>

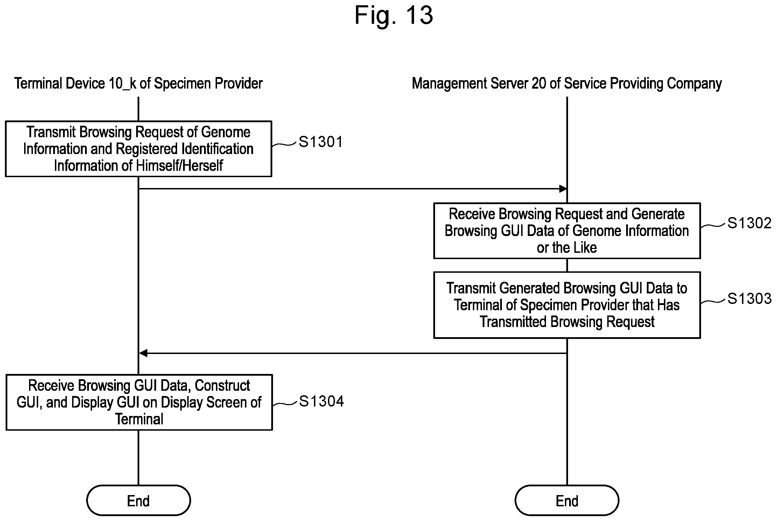

Fig. 13 is a flowchart for describing a browsing request process of these registered genome information and identification information of the specimen provider himself/herself executed between the terminal device 10_k (***k*** = 1 to ***n***) of the specimen provider and the management server 20 of the service providing company. Note that Fig. 13 gives a description assuming that the genome information database 100 is provided in the management server 20 of the service providing company, but the genome information database 100 may be provided in the management server 30 of the DB management company. In the latter case, the management server 20 and the management server 30 communicate with one another to transmit or receive information. In response to the browsing request by the specimen provider, the management server 20 of the service providing company acquires the genome information and the identification information registered in the genome information database 100 provided in the management server 30 of the DB management company, converts the information into browsing data, and provides the browsing data to the terminal device 10_k of the specimen provider.

### (i) Step 1301

The specimen provider starts the registration information browsing processing unit 1011 in the terminal device 10_k of the specimen provider and generates a browsing request of the information of himself/herself registered in the genome information database 100 using the input device 104. The registration information browsing processing unit 1011 transmits this browsing request to the management server 20 of the service providing company via the communication device 106. For example, when the specimen provider desires to confirm the registered genome information and identification information registered in the genome information database 100 and when the specimen provider desires to update or delete the identification information registered in in the past, the browsing request is transmitted to the management server 20. The browsing request preferably includes, for example, the identification information ("Individual Number", the genome information, the unique character string generated based on the genome information, and the like) of this specimen provider and the specimen provider name. This is because of ease of search for the information of this specimen provider from the genome information database 100.

### (ii) Step 1302

The genome information management unit 2012 in the management server 20 of the service providing company acquires the transmitted browsing request, acquires the genome information and the identification information of the target specimen provider based on this browsing request, and instructs the GUI generation processing unit 2014 to generate the browsing GUI data. The GUI generation processing unit 2014 generates the browsing GUI data based on a predetermined browsing format from the genome information and the identification information of the target specimen provider.

### (iii) Step 1303

The GUI generation processing unit 2014 transmits the generated browsing GUI data to the terminal device 10_k of the specimen provider who has transmitted the browsing request.

### (iv) Step 1304

The registration information browsing processing unit 1011 in the terminal device 10_k of the specimen provider acquires the browsing GUI data via the communication device 106, for example, constructs the browsing GUI data so as to fit to a browser, and displays the browsing GUI data on the screen of the output device (display device) 105.

By a sequence of the processes described above, the specimen provider can confirm the genome information obtained from the specimen of himself/herself and the provided identification information.

### <Genome Information Disclosure Process>

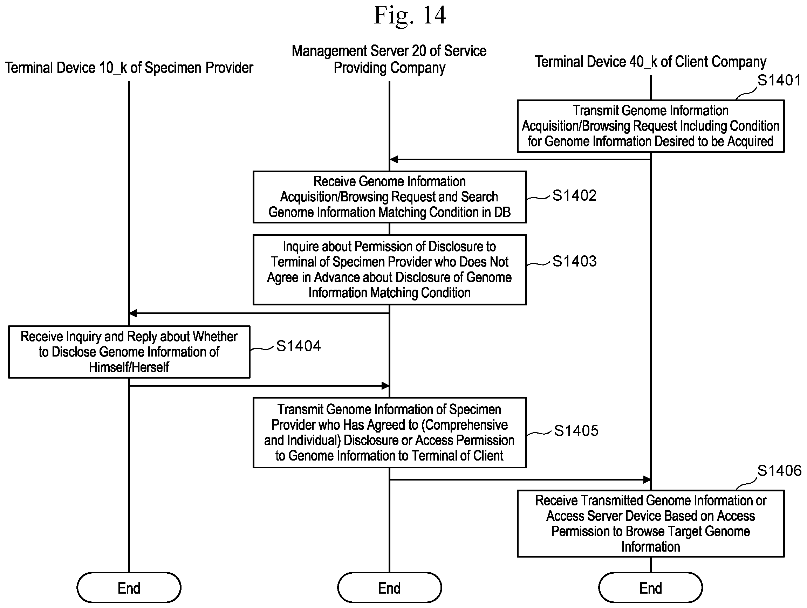

Fig. 14 is a flowchart for describing of a process that processes the genome information acquisition/browsing request from the terminal device 40_k (***k*** = 1 to ***n***) of the client company and discloses the desired genome information. Note that Fig. 14 gives a description assuming that the genome information database 100 is provided in the management server 20 of the service providing company, but the genome information database 100 may be provided in the management server 30 of the DB management company. In the latter case, the management server 20 and the management server 30 communicate with one another to transmit or receive information. In response to the genome information acquisition/browsing request by the client company, the management server 20 of the service providing company acquires the genome information and the identification information registered in the genome information database 100 provided in the management server 30 of the DB management company and gives access permission or transmits the acquired information to the terminal device 40_k of the client company.

### (i) Step 1401

The person in charge of the client company starts the genome information acquisition request processing unit 4011 in the terminal device 40_k of the client company and generates the genome information acquisition/browsing request including a condition (serving as a search condition) for the genome information desired to be acquired. In response to the input instruction by the person in charge of the client company, the genome information acquisition request processing unit 4011 transmits the generated genome information acquisition/browsing request to the management server 20 of the service providing company.

The "condition for the genome information desired to be acquired" included in the genome information acquisition/browsing request includes, for example, a sex, an age (range), a kind of illness, such as a cancer, a history of treatment, a state of disease, a case history, a family history, presence of a habit of drinking, and the like. The contents of this condition are optionally settable by the person in charge of the client company.

### (ii) Step 1402

When the management server 20 of the service providing company receives the genome information acquisition/browsing request from the terminal device 40_k of the client company, the genome information search processing unit 2013 starts, searches the genome information database 100 with the "condition for the genome information desired to be acquired" included in the genome information acquisition/browsing request as the search condition, and acquires the genome information matching the condition.

### (iii) Step 1403

The genome information management unit 2012 individually inquiries the permission (the intention of agreement) for the disclosure of the genome information to the terminal device 10_k (***k*** = 1 to ***n***) of the specimen provider who does not agree (including the comprehensive agreement and the agreement with condition (with condition: for example, a range of targets to be disclosed, a timing, a condition for utilization application, and the like)) among a plurality of specimen providers corresponding to the genome information matching the condition in advance. Note that the presence/absence of pre-agreement can be confirmed by checking the genome information database 100.

### (iv) Step 1404

When the terminal device 10_k of the specimen provider receives the inquiry of individual agreement from the management server 20 of the service providing company, in response to the input instruction (including whether to agree) by the specimen provider, the registration information disclosure agreement processing unit 1012 replies an answer for whether to disclose the genome information of this specimen provider and the like to the management server 20 of the service providing company.

### (v) Step 1405

When the management server 20 of the service providing company receives the answer for the inquiry from the terminal device 10_k of each specimen provider who has inquired, the genome information management unit 2012 gives access permission to the genome information of the specimen provider who has agreed the disclosure (comprehensive, with condition, and individual) to the terminal device 40_k of the client company who has issued the genome information acquisition/browsing request. For example, an access key is transmitted to this terminal device 40_k of the client company, and a predetermined memory area in the storage device 203 in the management server 20 is accessible with the access key, and thus the permission is given.

In addition to the access permission, the information matching the condition itself may be transmitted to the terminal device 40_k of the client company. In this case, the genome information management unit 2012 transmits the genome information matching the condition and to which the agreement has been made without change or processed into the browsing GUI data to the terminal device 40_k of the client company.

### (vi) Step 1406

When the terminal device 40_k of the client company receives the transmitted access permission of the genome information, the genome information acquisition/browsing processing unit 4012 accesses the predetermined storage area (stores the desired genome information or the like for browsing) in the storage device 203 in the management server 20 of the service providing company using the access key included in the access permission and displays the acquired genome information or the like on the display screen of the output device (display device) 405 of the terminal device 40_k of the client company. Alternatively, when the genome information or the like is directly transmitted, the genome information acquisition/browsing processing unit 4012 displays the received genome information or the like on the display screen of the output device (display device) 405.

A sequence of the processes described above allows the client company to acquire the desired genome information under the agreement by the specimen provider. Note that, as described above, for the client company to receive this service from the service providing company, the token needs to be purchased from a service provider in advance or when the genome information acquisition request is made.

### <Token Usage Process>

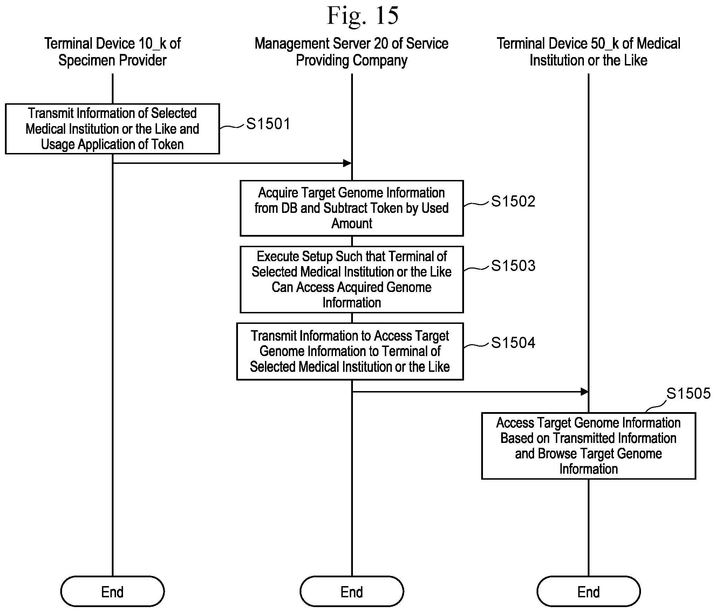

Fig. 15 is a flowchart for describing a process to use the token given to the specimen provider.

### (i) Step 1501

The specimen provider starts the token usage application processing unit 1014 from the terminal device 10_k (***k*** = 1 to ***n***)*,* selects the medical institution or the like (for example, a plurality of medical institutions or the like that have participated this token community) where the specimen provider desires to be examined and treated, and inputs the token usage application. In response to this input, the token usage application processing unit 1014 transmits the information of the selected medical institution or the like and the token usage application to the management server 20 of the service providing company.

### (ii) Step 1502

When the management server 20 of the service providing company receives the token usage application, the token management unit 2011 accesses the token information 1005 in the genome information database 100, subtracts the amount of used token, and replies the acceptance of the token usage application to the terminal device 10_k of the target specimen provider. In a case where the amount of possessed token is smaller than the amount of used token, the token management unit 2011 replies rejection of the token usage application and its reason to the terminal device 10_k of the target specimen provider.

### (iii) Step 1503

The genome information management unit 2012 executes setup such that the terminal device 50_k (***k*** = 1 to ***n***) of the medical institution or the like selected by the specimen provider can access the genome information of this specimen provider. For example, the genome information management unit 2012 stores the target genome information in the predetermined storage area in the storage device 203 and performs setting such that the access cannot be made without the access key.

### (iv) Step 1504

The genome information management unit 2012 transmits the access key for access to the genome information to the terminal device 50_k of the target medical institution or the like. Note that the genome information of the target specimen provider itself may be transmitted to the terminal device 50_k of the target medical institution or the like, or the access key may be transmitted to the terminal device 10_k of the target specimen provider, and the specimen provider presents the access key to the medical institution or the like to ensure the access.

### (v) Step 1505

When the terminal device 50_k of the medical institution or the like receives the access key for the genome information from the management server 20 of a service providing company, the registration information browsing processing unit 5012 accesses the predetermined memory area in the storage device 203 in the management server 20 of the service providing company based on this access key to access the target genome information, and displays the target genome information on the display screen of the output device (display device) 505. Note that when the target genome information itself is received, the genome information is directly displayed on the display screen.

### <Biobank Registration Process>

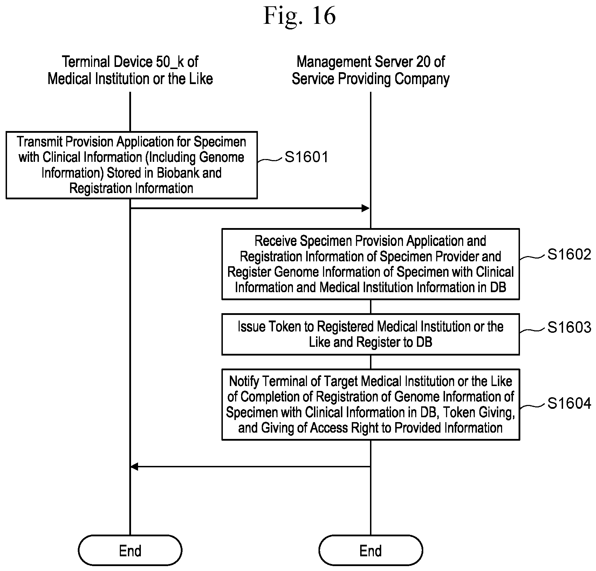

Fig. 16 is a flowchart for describing a registration process of the specimen with clinical information (includes the genome information when the specimen analysis has been performed and the genome information is present) stored in the biobank by the medical institution or the like in the genome information database 100. Note that Fig. 16 gives a description assuming that the genome information database 100 is provided in the management server 20 of the service providing company, but the genome information database 100 may be provided in the management server 30 of the DB management company. In the latter case, the management server 20 and the management server 30 communicate with one another to transmit or receive information. The genome information obtained through the analysis of the specimen acquired from the medical institution or the like by the management server 20 of the service providing company, the clinical information, and the like are registered in the genome information database 100 provided in the management server 30 of the DB management company.

### (i) Step 1601

The person in charge of the medical institution or the like accesses (for example, accesses the service site of the service providing company) the management server 20 of the service providing company using the communication device 506 of the terminal device 50_k (***k*** = 1 to ***n***) of the medical institution or the like and downloads a specimen with clinical information provision application form. The person in charge starts the specimen/clinical information provision processing unit 5011, inputs an intention of the application for the provision of the specimen with clinical information stored in the biobank to the application form using the input device 504, and transmits the form to the management server 20 of the service providing company using the communication device 506. At this time, the terminal device 50_k of the medical institution or the like receives a reception number (the information with which the fact of the provision by this medical institution or the like can be uniquely identified) from the management server 20 and stores the reception number in the memory 502 or the storage device 503.

For example, after the person in charge of the medical institution or the like receives the reception number, the person in charge delivers the specimen with clinical information stored in the biobank to the service providing company together with this reception number. Alternatively, the person in charge delivers it to an inspection center or the like where the genome analysis is performed. Note that the clinical information may be transmitted to the management server 20 of the service providing company over the network 60 separately from the specimen.

### (ii) Step 1602

The management server 20 of the service providing company receives the specimen with clinical information provision application from the terminal device 50_k of the medical institution or the like. After the reception of the specimen with clinical information delivered from the target medical institution or the like is confirmed, in response to the instruction of the registration of the specimen provision application or the like input by the person in charge of the service providing company, the genome information management unit 2012 makes the clinical information and the genome information obtained through the analysis of the specimen correspond to the identification information of the target medical institution or the like, and registers it in the genome information database 100. Since the genome information is obtained through the analysis of the specimen, in a case where the genome information is absent at the time point of the acceptance of the provision application, the genome information is not registered in the genome information database 100 at this phase. However, in a case where the genome information is attached to the provided specimen, the genome information may be immediately registered in the genome information database 100, or the genome information may be temporarily registered, and after the specimen analysis by the service providing company is terminated, the temporarily registered genome information may be switched to the genome information obtained through this specimen analysis.

After the analysis of the specimen provided to the service providing company is terminated, the genome information management unit 2012 registers the analysis result in the genome information database 100 as the genome information.

### (iii) Step 1603

The token management unit 2011 in the management server 20 issues the token to the medical institution or the like that has provided the specimen with clinical information, and registers the amount of issued token in the token information 1005 in the genome information database 100. The biobank is a facility and a system that stores the specimens and the genome information of various patients together with the clinical information. The biobank stores an enormous volume of specimens and information. Therefore, a necessity of changing the amount of token given depending on the level of detail of the information as in the case where the specimen and the identification information are provided by specimen provider as described above is small, and the predetermined amount of token can be equally given. Additionally, when the personal information (such as a disease and a progress of treatment) is added to and updated in the biobank, additionally giving the token is also possible.

### (iv) Step 1604

The genome information management unit 2012 notifies the terminal device 50_k of this medical institution or the like of the completion of registration of the information in the biobank of the medical institution or the like (the clinical information, the specimen, the genome information, and the detailed information of the provider (patient) of each specimen) in the genome information database 100, the token grant, and the completion of registration in the genome information database 100 (token information 1005). In the case of the medical institution or the like, unlike the above-described specimen provider, intention confirmation regarding the disclosure of the genome information or the like is not performed. This is because the medical institution or the like provides the specimen with clinical information on the assumption of the disclosure.

The genome information management unit 2012 gives an access right to the provided specimen with clinical information and genome information to the terminal device 50_k of the medical institution or the like and notifies the fact.

### <Process of Registration Information Browsing Request by Medical Institution or the Like>

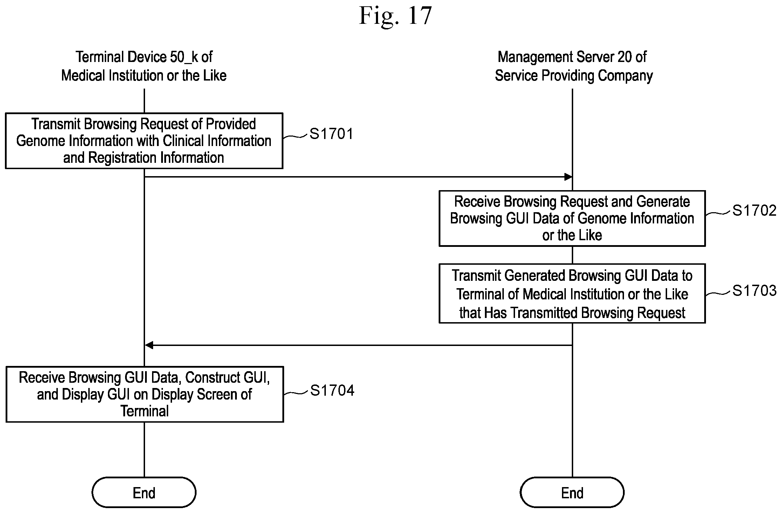

Fig. 17 is a flowchart for describing a process when the medical institution or the like transmits a browsing request of the specimen with clinical information and the genome information registered in the genome information database 100 to the management server 20 of the service providing company. Note that Fig. 17 gives a description assuming that the genome information database 100 is provided in the management server 20 of the service providing company, but the genome information database 100 may be provided in the management server 30 of the DB management company. In the latter case, the management server 20 and the management server 30 communicate with one another to transmit or receive information. In response to the browsing request by the medical institution or the like, the management server 20 of the service providing company acquires the specimen with clinical information and the genome information registered in the genome information database 100 provided in the management server 30 of the DB management company, converts the information into the browsing data, and provides the browsing data to the terminal device 50_k of the medical institution or the like.

### (i) Step 1701

The person in charge of the medical institution or the like starts the registration information browsing processing unit 5012 in the terminal device 50_k of the medical institution or the like and generates the browsing request of the specimen with clinical information or the like registered in the genome information database 100 using the input device 504. The registration information browsing processing unit 5012 transmits this browsing request to the management server 20 of the service providing company via the communication device 506. For example, the browsing request is transmitted to the management server 20 when the person in charge of the medical institution or the like desires to confirm the registered specimen with clinical information and genome information registered in the genome information database 100, and desires to update or delete the information registered in the past (for example, when a patient as the provider of the registered specimen has died or the like). The browsing request preferably includes, for example, the identification information of this medical institution or the like and the name of the medical institution or the like. This is because the information of this medical institution or the like is easily searched from the genome information database 100.

### (ii) Step 1702

The genome information management unit 2012 in the management server 20 of the service providing company acquires the transmitted browsing request, acquires the registration information (the specimen with clinical information and the genome information) of the target medical institution or the like based on this browsing request from the genome information database 100, and instructs the GUI generation processing unit 2014 to generate the browsing GUI data. The GUI generation processing unit 2014 generates the browsing GUI data based on the predetermined browsing format from the registration information of the target medical institution or the like.

### (iii) Step 1703

The GUI generation processing unit 2014 transmits the generated browsing GUI data to the terminal device 50_k of the medical institution or the like that has transmitted the browsing request.

### (iv) Step 1704

The registration information browsing processing unit 5012 in the terminal device 50_k of the medical institution or the like acquires the browsing GUI data via the communication device 506, constructs the browsing GUI data, for example, so as to fit to the browser, and displays the browsing GUI data on the screen of the output device (display device) 505.

A sequence of the processes described above allows the medical institution or the like to confirm the genome information obtained from the provided specimen and the provided identification information.

### <Clinical Trial Collaboration Request Process>

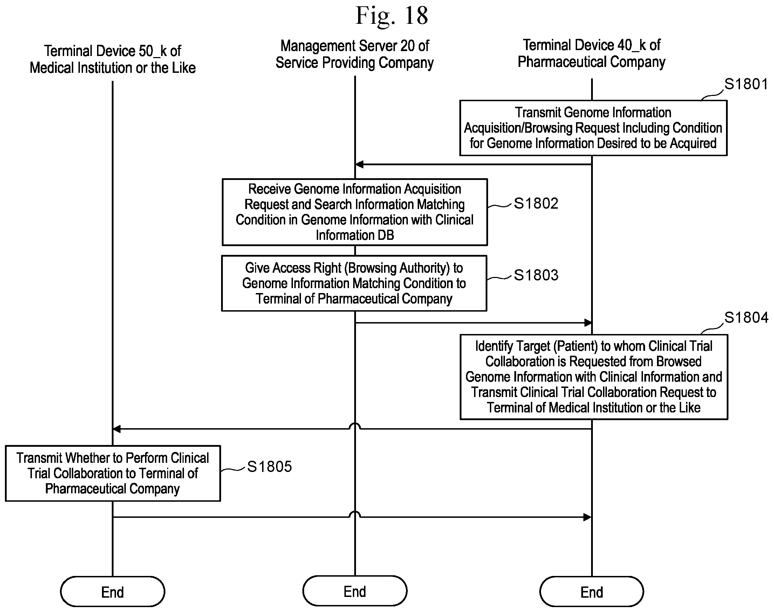

Fig. 18 is a flowchart for describing a sequence of processes when the client company (pharmaceutical company) acquires the desired genome information from the service providing company and makes the clinical trial collaboration request to the target medical institution or the like. Note that Fig. 18 gives a description assuming that the genome information database 100 is provided in the management server 20 of the service providing company, but the genome information database 100 may be provided in the management server 30 of the DB management company. In the latter case, the management server 20 and the management server 30 communicate with one another to transmit or receive information. In response to the genome information acquisition/browsing request by the pharmaceutical company, the management server 20 of the service providing company acquires the genome information and the identification information registered in the genome information database 100 provided in the management server 30 of the DB management company, and gives an access permission or transmits the acquired information to the terminal device 40_k of the pharmaceutical company.

### (i) Step 1801

The person in charge of the pharmaceutical company starts the genome information acquisition request processing unit 4011 in the terminal device 40_k of the pharmaceutical company and generates the genome information acquisition/browsing request including the condition (serving as the search condition) for the genome information desired to be acquired. In response to the input instruction by the person in charge of the pharmaceutical company, the genome information acquisition request processing unit 4011 transmits the generated genome information acquisition/browsing request to the management server 20 of the service providing company.

The "condition for the genome information desired to be acquired" included in the genome information acquisition/browsing request includes, for example, a sex, an age (range), a kind of illness, such as a cancer, a history of treatment, a state of disease, a case history, a family history, presence of a habit of drinking, and the like. The contents of this condition are optionally settable by the person in charge of the client company.

Additionally, the person in charge of the pharmaceutical company may check the registered biobank provided from the service providing company and the information of the medical institution or the like that possesses the biobank, and one or more of the medical institutions or the like among them may be the destination of the genome information acquisition/browsing request.

### (ii) Step 1802

When the management server 20 of the service providing company receives the genome information acquisition/browsing request from the terminal device 40_k of the pharmaceutical company, the genome information search processing unit 2013 starts, searches the genome information database 100 with the "condition for the genome information desired to be acquired" or "designated medical institution or the like" included in the genome information acquisition/browsing request as the search condition, and acquires the information of the specimen with clinical information, the genome information, or the like matching the condition.

### (iii) Step 1803

The genome information management unit 2012 gives an access permission (access right) to the information of the specimen with clinical information and the genome information acquired by the search to the terminal device 40_k of the pharmaceutical company that has output the genome information acquisition/browsing request. For example, an access key is transmitted to the terminal device 40_k of this pharmaceutical company, and a predetermined memory area in the storage device 203 in the management server 20 is accessible with the access key, and thus the access permission (access right) is given.

In addition to the access permission, the information matching the condition itself may be transmitted to the terminal device 40_k of the pharmaceutical company. In this case, the genome information management unit 2012 directly transmits the information of the specimen with clinical information and the genome information matching the condition, or processes the information into the browsing GUI data and transmits the browsing GUI data to the terminal device 40_k of the pharmaceutical company.

### (vi) Step 1804

When the terminal device 40_k of the pharmaceutical company receives the transmitted access permission (access right), the genome information acquisition/browsing processing unit 4012 accesses the predetermined storage area (stores the desired genome information with the clinical information or the like for browsing) in the storage device 203 in the management server 20 of the service providing company using the access key included in the access permission, and displays the acquired genome information with the clinical information or the like on the display screen of the output device (display device) 405 in the terminal device 40_k of the client company. When the genome information or the like is directly transmitted, the genome information acquisition/browsing processing unit 4012 displays the received genome information or the like on the display screen of the output device (display device) 405.

When the genome information acquisition/browsing request transmitted from the pharmaceutical company to the service providing company includes the designation of the medical institution or the like, in response to the input instruction by the person in charge of the pharmaceutical company, the clinical trial collaboration request handling unit 5013 transmits the clinical trial collaboration request to the terminal device 50_k (***k*** = 1 to ***n***) of this designated medical institution or the like.

On the other hand, when the genome information acquisition/browsing request transmitted from the pharmaceutical company to the service providing company does not include the designation of the medical institution or the like, the acquired/browsed genome information with clinical information is more likely to be acquired from a plurality of medical institutions or the like. In this case, for example, when the person in charge of the pharmaceutical company identifies (selects) the targets (patients) to whom the clinical trial collaboration is requested from the genome information with clinical information or the like browsed on the displayed GUI, the genome information acquisition/browsing processing unit 4012 displays a list of the medical institutions or the like where these respective identified targets (patients) are examined and treated (or were examined and treated) on the screen. When the person in charge of the pharmaceutical company selects the medical institution or the like to which the clinical trial collaboration request has been made from this list, the clinical trial collaboration request handling unit 5013 transmits the clinical trial collaboration request to the terminal device 50_k (***k*** = 1 to ***n***) of the selected medical institution or the like. The clinical trial collaboration request can be transmitted to a plurality of medical institutions or the like.

### (v) Step 1805

When the terminal device 50_k of the medical institution or the like receives the clinical trial collaboration request from the terminal device 40_k of the pharmaceutical company, whether to accept the clinical trial collaboration is discussed in the medical institution. In response to the input instruction by the person in charge of this medical institution or the like, the clinical trial collaboration request handling unit 5013 replies an answer for the clinical trial collaboration request (whether to perform clinical trial collaboration) to the terminal device 40_k of the pharmaceutical company.

The handling for the clinical trial collaboration request may be preliminarily determined, the answer to the clinical trial collaboration request may be automatically generated by the clinical trial collaboration request handling unit 5013, and the answer may be transmitted to the terminal device 40_k of the pharmaceutical company.

### <Summary>

(i) In this embodiment, a distinctive configuration of the server device (the management server 20 of the service providing company) will be disclosed. The management server 20 of the service providing company is the server device that provides the genome related services. The management server 20 executes a process that generates information that can be exchanged for value (token) for predetermined settlement given to the specimen provider as the compensation for providing the specimen, associates the genome information obtained through the analysis of the provided specimen and the identification information (for example, "Individual Number", the genome information, the unique character string generated based on the genome information, and the like) of the specimen provider with the generated information that can be exchanged for value (sets index data such that all pieces of the information are associated with "Individual Number" or the like), and stores the associated information in the database (genome information database 100). This allows giving an incentive brought by providing the specimen to many people, thereby allowing efficiently collecting and effectively utilizing the genome information. Note that this genome information database 100 needs not to be provided in the management server 20 and may be provided in the management server 30 or the like. To speak further, the management server 20 and the management server 30 only need to include at least the database components equivalent to Fig. 7, and the other database components (equivalent to Fig. 8 to Fig. 11) may be provided in the management server 20 and the management server 30 or may be physically present on the network as other server devices. However, an important thing here is that various pieces of information and data constituted from Fig. 7 to Fig. 11 stored in the genome information database 100 are linked with the identification information (for example, "Individual Number") of the specimen provider to be managed.

The genome information database 100 associates the identification information other than the genome information of the specimen provider (at least one of general information of the specimen provider, past medical history information, case history information of family, information related to lifestyle habits, treatment/progress information of illness, proteomics information, or metabolomics information) with the identification information of the specimen provider, and stores the associated information. When the genome information is utilized, the genome information can be associated with the identification information to be analyzed. Accordingly, for example, association of the features of the specimen provider (example: the previous illness and the lifestyle habits), the currently affected illness, and the genome information is examined, and it can be useful to develop a new medicine (discover a drug) and develop a new diagnostic method. Note that the past and current genome information or the like are associated with the identification information with which the specimen provider can be uniquely identified and registered in the genome information database 100. This allows a comparison between the past genome information or the like and the current genome information or the like of this specimen provider, and therefore the genome information can be further effectively analyzed.

When the genome information of the specimen provider is utilized, the management server 20 associates the utilization history information (information of a person who utilizes the genome information and information of utilization timing) including at least utilization contents with the identification information of the specimen provider, and stores the associated information in the genome information database 100. From a viewpoint of the specimen provider, browsing the genome information of himself/herself allows confirming and realizing that how the genome information of himself/herself is utilized and how the genome information can be contributed to the society. From a viewpoint of the person who performs utilization, accumulation of the utilization history information allows knowing a trend of the frequently utilized genome information, conversely a trend of the genome information not utilized at all, or the like.

The management server 20 receives the request (genome information acquisition/browsing request) including the desired condition of the genome information desired to be acquired from the computer of the utilization requester of the genome information (client company: pharmaceutical company) over the network, searches the genome information database 100, identifies the specimen provider matching the desired condition, and transmits the genome information of this specimen provider to the computer of the client company, or permits an access to the genome information from the computer of the client company. Note that this genome information is provided or the access permission to this genome information is given on the condition of the purchase of the information that can be exchanged for value (token) by the utilization requester (client company) (in advance) and the presentation of the information that can be exchanged for value (at the genome information acquisition). By making a large number pieces of valuable genome information into the database, an increase in companies that desire to utilize the genome information can be expected. Additionally, introducing the economically added value, the token, allows smoothly advancing a cycle of the provision of the specimen, the accumulation of the genome information generated by analyzing the specimen, and the effective utilization of the genome information.

It is assumed that the genome information is not provided (acquired or browsable) to the client company with no condition, but the specimen provider agrees to the disclosure. Specifically, the inquiry of whether to agree to the provision of the genome information of himself/herself is transmitted to the computer of the specimen provider matching the desired condition, only the genome information of the specimen provider from whom the declaration of the intention of the agreement is received is transmitted to the computer of the client company, or the computer of the client company is permitted to access only the agreed genome information. This allows guaranteeing safety to the disclosure of the information of the specimen provider. The agreement includes the individual agreement that shows the declaration of intention of the presence/absence of agreement whenever the client company makes a genome information acquisition/browsing request and the comprehensive agreement that shows the declaration of intention of agreement to all disclosures when the specimen provider provides the specimen.

Furthermore, the management server 20 checks whether the specimen provider who provided the specimen and the identification information has previously provided the specimen and the identification information. When the specimen provider has provided the specimen or the like previously, the management server 20 associates the current information with the previous information using the identification information of this specimen provider and registers the associated information in the genome information database 100. Accordingly, all of the current and past genome information and identification information of this specimen provider can be searched using the identification information as the key, and this allows analyzing a change over time of the genome information of the same specimen provider.

(ii) The functions of these embodiments can also be achieved by program codes of software. In this case, a storage medium recording the program codes are provided to the system or the device, and a computer (or a CPU and an MPU) in the system or the device reads the program codes stored in the storage medium. In this case, the program codes themselves read from the storage medium achieve the above-described functions of the embodiments. The program codes themselves and the storage medium storing the program codes configure these embodiments. As examples of the storage medium supplying such program codes, a flexible disk, a CD-ROM, a DVD-ROM, a hard disk, an optical disk, a magneto-optical disk, a CD-R, a magnetic tape, a non-volatile memory card, a ROM, and the like are used.

An operating system (OS) operating on the computer or the like may execute a part of or all of the actual processes based on instructions from the program codes, and the above-described functions of the embodiments may be achieved by the processes. Furthermore, after the program codes read from the storage medium are written to a memory in the computer, the CPU in the computer or the like may execute a part of or all of the actual processes based on the instructions from the program codes, and the above-described functions of the embodiments may be achieved by the processes.

Furthermore, the program codes of the software, which achieve the functions of the embodiments, may be distributed over a network, storage means, such as a hard disk and a memory in the system or the device, or the storage medium, such as a CD-RW and a CD-R, may store the program codes, and the computer (or the CPU and the MPU) in the system or the device may read and execute the program codes stored in this storage means and this storage medium for use.

Finally, it is necessary to understand that the processes and techniques described here are not essentially related to any particular device and can be mounted by any suitable combination of components. Furthermore, general-purpose, various types of devices can be used in accordance with the teaching described here. To execute the steps of the method described here, it may be found that constructing a dedicated device is beneficial. Further, various kinds of inventions can be made by properly combining the plurality of components disclosed in the embodiments. For example, some components may be omitted from all of the components shown in the embodiments. Moreover, components in different embodiments may be suitably combined together. While these embodiments have been described related to the specific examples, these examples are not limited in all aspects but are for description. A person skilled in this field probably understands that a large number of combinations of hardware, software, and firmware suitable to embody these embodiments are present. For example, the described software can be implemented by wide-range programs or script languages, such as assembler, C/C++, perl, Shell, PHP, Java (registered trademark), and a blockchain-related programming language (such as Solidity, Serpent, Viper, and LLL).

Furthermore, in the above-described embodiments, control lines and information lines considered necessary for the description are described. Not all the control lines or the information lines for the product are necessarily described. All configurations may be mutually coupled.

This disclosure is not limited to the above-described embodiments but includes various modifications. The above-described embodiments have been described in detail for ease of understanding of this disclosure and are not necessarily limited to one that includes all the described configurations.

### Reference Signs List

- 1: Service providing system
- 10_1 to 10_n (10_k): Terminal device of specimen provider
- 20: Management server of service providing company
- 30: Management server of DB management company
- 40_1 to 40_n: Terminal device of client company
- 50_1 to 50_n: Terminal device medical institution or the like
- 60: Network

## Claims

1. A server device that provides a genome related service, the server device comprising:
a storage device that stores a program to provide the service; and
a processor that reads the program from the storage device and executes the program,
wherein the processor executes:
a process that generates information that can be exchanged for value for predetermined settlement given to a specimen provider as a compensation for providing a specimen; and
a process that associates genome information obtained through an analysis of the provided specimen and identification information of the specimen provider with the generated information that can be exchanged for value and stores the associated information in a database.

2. The server device according to claim 1,
wherein the processor associates identification information other than the genome information of the specimen provider with the identification information of the specimen provider and stores the associated information in the database.

3. The server device according to claim 2,
wherein the identification information includes at least one of general information of the specimen provider, past medical history information, case history information of family, information related to a lifestyle habit, treatment/progress information of an illness, proteomics information, or metabolomics information.

4. The server device according to any one of claims 1 to 3,
wherein when the genome information of the specimen provider is utilized, the processor executes a process that associates utilization history information including at least utilization contents with the identification information of the specimen provider and stores the associated information in the database.

5. The server device according to claim 4,
wherein the utilization history information includes information of a person who utilizes the genome information and information of a utilization timing.

6. The server device according to any one of claims 1 to 5,
wherein the processor executes a process that generates a user interface to allow the specimen provider to browse the genome information and the information that can be exchanged for value of himself/herself.

7. The server device according to any one of claims 1 to 6,
wherein the processor further executes:
a process that receives a request including a desired condition for genome information desired to be acquired from a computer of a utilization requester of the genome information over a network;
a process that searches the database based on the desired condition included in the request and identifies the specimen provider matching the desired condition; and
a process that transmits the genome information of the specimen provider matching the desired condition to the computer of the utilization requester, or a process that permits an access to the genome information of the specimen provider matching the desired condition from the computer of the utilization requester.

8. The server device according to claim 7,
wherein the processor transmits the genome information to the computer of the utilization requester, or permits the access to the genome information from the computer of the utilization requester on a condition that the utilization requester purchases the information that can be exchanged for value.

9. The server device according to claim 7 or 8,
wherein the processor transmits an inquiry of whether to agree to the provision of the genome information of himself/herself to a computer of the specimen provider matching the desired condition, and the processor transmits the genome information of the specimen provider from whom a declaration of an intention of the agreement has been received to the computer of the utilization requester, or permits an access to the genome information of the specimen provider from whom the declaration of the intention of the agreement has been received from the computer of the utilization requester.

10. The server device according to claim 7 or 8,
wherein the processor, without transmitting an inquiry of whether to make an agreement again regarding the genome information of the specimen provider matching the desired condition of the specimen provider who has preliminarily shown a declaration of an intention of comprehensive agreement to the provision of the genome information of himself/herself to the computer of the specimen provider, transmits the genome information to the computer of the utilization requester or permits an access to the genome information from the computer of the utilization requester.

11. The server device according to any one of claims 1 to 10,
wherein the processor further executes:
when the specimen or identification information is provided from the specimen provider, a process that searches the database using the identification information of the specimen provider and determines whether the specimen or the identification information has been provided from the specimen provider at a first time point; and
when the specimen or the identification information has been provided from the specimen provider at the first time point, a process that associates the genome information or the identification information at the first time point with genome information or identification information at a second time point temporally newer than the genome information or the identification information at the first time point, and stores the associated information in the database.

12. A service providing system comprising:
a server device that provides a genome related service;
a first computer of a specimen provider that provides a specimen to collect genome information; and
a second computer of a utilization requester who desires to utilize the genome information,
wherein the server device, the first computer, and the second computer are connected over a network,
wherein (i) the server device executes:
a process that generates information that can be exchanged for value for predetermined settlement given to the specimen provider as a compensation for providing the specimen;
a process that associates the genome information obtained through an analysis of the provided specimen and identification information of the specimen provider with the generated information that can be exchanged for value and stores the associated information in a database;
a process that acquires the genome information of the specimen provider and the given information that can be exchanged for value from the database in response to a browsing request of the genome information of the specimen provider received from the first computer and allows the specimen provider to browse the acquired information;
a process that receives a genome information acquisition request including a desired condition of desired genome information received from the second computer;
a process that searches the database based on the desired condition included in the genome information acquisition request and identifies the specimen provider matching the desired condition; and
a process that transmits the genome information of the specimen provider matching the desired condition to the second computer or a process that permits an access to the genome information of the specimen provider matching the desired condition from the second computer,
wherein (ii) the first computer executes:
a process that transmits the browsing request to the server device; and
a process that displays the genome information of the specimen provider and the information that can be exchanged for value on a display screen of the first computer, and
wherein (iii) the second computer executes:
a process that transmits the genome information acquisition request to the server device; and
a process that acquires the genome information of the specimen provider matching the desired condition from the server device, or a process that accesses the server device to access the genome information of the specimen provider matching the desired condition.

13. The service providing system according to claim 12,
wherein the server device associates identification information other than the genome information of the specimen provider with specimen information of the specimen provider and stores the associated information in the database.

14. The service providing system according to claim 13,
wherein the identification information includes at least one of general information of the specimen provider, past medical history information, case history information of family, information related to a lifestyle habit, treatment/progress information of an illness, proteomics information, or metabolomics information.

15. The service providing system according to any one of claims 12 to 14,
wherein when the genome information of the specimen provider is utilized, the server device executes a process that associates utilization history information including at least utilization contents with the identification information of the specimen provider and stores the associated information in the database.

16. The service providing system according to claim 15,
wherein the utilization history information includes information of a person who utilizes the genome information and information of a utilization timing.

17. The service providing system according to any one of claims 12 to 16,
wherein the server device transmits the genome information to the second computer, or permits the access to the genome information from the second computer on a condition that the utilization requester purchases the information that can be exchanged for value.

18. The service providing system according to any one of claims 12 to 17,
wherein the server device transmits an inquiry of whether to agree to the provision of the genome information of the specimen provider to the first computer matching the desired condition, and the server device transmits only the genome information of the specimen provider from whom a declaration of an intention of the agreement is received to the second computer, or permits the second computer to access the genome information of the specimen provider from whom the declaration of the intention of the agreement is received.

19. The service providing system according to any one of claims 12 to 18,
wherein the server device, without transmitting an inquiry of whether to make an agreement again regarding the genome information of the specimen provider matching the desired condition of the specimen provider who has preliminarily shown a declaration of an intention of comprehensive agreement to the provision of the genome information of himself/herself to the first computer, transmits the genome information to the second computer or permits the access to the genome information from the second computer.

20. The service providing system according to any one of claims 12 to 18,
wherein the server device further executes:
when the specimen or identification information is provided from the specimen provider, a process that searches the database using the identification information of the specimen provider and determines whether the specimen or the identification information has been provided from the specimen provider at a first time point; and
when the specimen or the identification information has been provided from the specimen provider in a past, a process that associates the genome information or the identification information at the first time point with genome information or identification information at a second time point temporally newer than the genome information or the identification information at the first time point, and stores the associated information in the database.
